# EUROPEAN PATENT APPLICATION

(11) **EP 1 647 181 A1**
(43) Date of publication of application: **19.04.2006**
(21) Application number: 04747840.9
(22) Date of filing: 15.07.2004
(51) Int. Cl.: A01H 4/00, C12N 15/09, C12N 5/14

(54) **METHOD OF CONSTRUCTING PLANT SHOWING IMPROVED GROWTN UNDER REGULATION OF NITROGEN**

(30) Priority: 17.07.2003 JP 2003198559
(71) Applicant: Ajinomoto Co., Inc., Tokyo 104-8315 (JP)
(72) Inventor: KISAKA, Hiroaki; c/o AJINOMOTO CO., INC., Kawasaki-shi, Kanagawa 2108681 (JP); MIWA, Tetsuya; c/o AJINOMOTO CO., INC., Kawasaki-shi, Kanagawa 2108681 (JP); AKIYAMA, Ai; c/o AJINOMOTO CO., INC., Kawasaki-shi, Kanagawa 2108681 (JP)
(74) Representative: HOFFMANN EITLE
(86) International application number: PCT/JP2004/010451
(87) International publication number: WO 2005/006847

(57) **Abstract**

The present invention provides a method of producing a plant exhibiting improved growth and/or yield under conditions where nitrogen is reduced, that is, under cultivation conditions where nitrogen is limited compared as the ordinary cultivation conditions, by increasing 2-OG content in plants. Particularly, The present invention provides a method of producing a plant exhibiting improved growth and/or yield under conditions where nitrogen is reduced, that is, under cultivation conditions where nitrogen is limited compared as the ordinary cultivation conditions, by introducing a GDH gene or ECASPC gene into plants and expressing the transgene GDH or ECAPS in the plants to increase 2-OG or by spraying proline on the leaves of plants to increase 2-OG content, thereby enhancing the incorporation of nitrogen or metabolic activity of plants. Also provided is a method of cultivating such plants under nitrogen-limited conditions.

## Description

### Filed of the Invention

The present invention relates to a method for improving the growth and yields of plants under nitrogen-limited conditions.

### Backctround of the Invention

Application of chemical fertilizers to supply nitrogen as observed in modern intensive agriculture results in dramatic improvements in yield, but release of nitrogen into the surrounding environment in the form of excess nitrates raised such environmental problems as contamination of rivers and lakes by eutrophication, health hazards from accumulation of nitrates in crops, and generation of greenhouse gases from residual nitrogen in the soil. The large amount of electricity required for manufacturing nitrogen fertilizers also contributes to the environmental burden of agriculture. In other words, agricultural methods that use less chemical fertilizer, particularly nitrogen fertilizer, and less nitrogen fertilizer are necessary in order to achieve sustainability.

However, with a few exceptions such as the *Leguminosae,* plants cannot fix nitrogen from the air, and are entirely dependent for nitrogen on nitrate or ammonia supplied from outside. Consequently, nitrogen is the greatest limiting factor on the growth and development of plants, and modern agriculture is faced with the apparently conflicting tasks of maintaining and increasing current yields while reducing the aforementioned environmental burden in an effort towards sustainability. Efforts in this direction, and in particular efforts to develop and cultivate plants with satisfactory growth and yields under conditions of reduced nitrogen supply have so far been inadequate. For example, environment-friendly and sustainable organic agriculture has been proposed as an alternative to modern intensive agriculture, but while application of nitrogen fertilizer is controlled in such environment-friendly organic agriculture, there have been many problems in terms of yield.

In terms of improving plant cultivars, there have been no successful examples of plant breeding to solve these issues by providing adequate growth and/or yields under nitrogen-limited conditions such as the conditions where the application of nitrogen fertilizer is reduced. Consequently, there is a demand for the breeding of plant cultivars having maintained or improved yields by modifying or improving the utilization efficiency of nitrogen by plants under less application of nitrogen fertilizer, and for the development of technologies for breeding such plant cultivars.

The first stage of assimilation of inorganic nitrogen into the organic form in plants is known to be mainly incorporation of ammonia into glutamate to produce glutamine. This is a reaction catalyzed by glutamine synthetase (GS) and glutamate synthase (GOGAT), in which the two reactions combine to produce one molecule of glutamate by assimilation of one molecule of ammonia into one molecule of 2-OG. This GS/GOGAT cycle is thought to be the primary pathway of nitrogen assimilation in plants (Miflin and Lea: 1976, *Phytochemistry* 15: 873-885).

In microorganisms, on the other hand, it has been reported from studies using *Cyanobacteria* and *E*. *coli* that the quantitative balance of 2-OG and glutamine regulates the expression of enzyme genes associated with incorporation and assimilation of nitrate (Forchammer and Tandeau-Marsac, 1995, *J*. *Bacteriol* 177, 2033-2040; Jiang et al, 1998, *Biochemistry* 37, 12795-12801). However a gene has been isolated from *Arabidopsis thaliana* (Arabidopsis) having strong homology with the PII protein, which is thought to be associated with such regulation, and when this gene was introduced and over-expressed in tobacco no increase in nitrate content was seen, suggesting that accumulation of nitrogen in higher plants occurs differently as compared with in microorganisms (Hsieh et al, 1998, *Proc. Natl. Acad. Sci. USA* 95: 13965-13970). On the other hand, it has been reported that when expression of nitrate reductase (hereunder abbreviated as NR) was investigated using tobacco plants having the ferredoxin-dependent glutamate synthase gene introduced in the antisense direction so as to reduce expression of glutamate synthase, the transcription level of NR increased when nitrate, sucrose and 2-OG were supplied, while when glutamine was supplied the NR transcription level decreased (Ferrario-Mery et al, 2001 *Planta* 213: 265-271). According to this reference, the amounts of 2-OG and glutamine are also associated with regulation of NR in higher plants. However, these findings only relate to NR, and nothing has been said about the effects of 2-OG on the growth and development of plants under nitrogen-limited conditions. Moreover, 2-OG is a relatively unstable compound and has been considered to be impractical for, for example, spraying purposes.

Transgenic plants into which a GDH gene has been introduced have also been developed, and it has been reported that when an *E. coli*-derived NADP-dependent GDH gene (gdhA) was introduced into tobacco and corn with the aim of conferring resistance to the herbicide phosphinothricin, dried weight, total amino acid content and water-soluble carbohydrate content all increased significantly (Lightfoot et al, CA2180786, 1996). Similarly, Tian et al (CN00109779.2) introduced a *Neurospora*-derived NADP-dependent GDH gene into tobacco and reported that growth was better than that of tobacco into which the gene was not introduced. It has also been reported that free amino acid content in the fruit increased when an *Aspergillus nidulans*-derived NADP-dependent GDH gene was introduced into tomato plants (Kisaka et al, JP11-376710) and that tuber weight and number of tubers increased when the same gene was introduced into potato plants (Kisaka et al, JP2000-404322). However, the function of the introduced gene is not elucidated in any of these reports, and it is unknown how the introduced gene functions or what effects produce these characteristics. Moreover, nothing is mentioned about improving the growth and/or yields of plants under nitrogen-limited conditions.

There have also been no reports regarding introduction of the aspartate aminotransferase gene into plants.

### Summary of the Invention

It is an object of the present invention to provide a method for developing plants having enhanced nitrogen incorporation and enhanced metabolic activity of plants and having improved growth and/or yields under cultivation conditions of limited nitrogen or in other words with nitrogen limited to the level below the normal cultivation conditions, as well as a method of cultivating such plants under nitrogen-limited conditions.

The inventors assumed the existence of a signal molecule that allows plant cells to sense nitrogen nutrient conditions, and considered 2-oxoglutarate (2-OG) as such a signal molecule. That is, 2-OG is a signal molecule which comprehensively regulates nitrogen incorporation and metabolism in plant cells, and it was thought that by artificially increasing and decreasing the content of this substance it would be possible to maintain nitrogen incorporation and metabolism in an elevated state in plant cells. Moreover, because a plant in such an elevated state is believed to be capable of actively incorporating nitrogen from exterior, it was thought that nitrogen would be replenished under nitrogen-limited conditions, thereby improving growth and yield. Moreover, the inventors discovered that the 2-OG content of plant cells could be increased by causing over-expression of glutamate dehydrogenase (hereunder abbreviated as GDH) activity or aspartate aminotransferase (hereunder abbreviated as ASPC) activity, and that plants with increased 2-OG content have improved growth and/or yields under nitrogen-limited conditions, which led to the establishment of the present inventions.

Consequently, the present invention is a method of producing a plant exhibiting improved growth and/or yield under cultivation conditions where nitrogen is limited to the level below normal cultivation conditions, by increasing the 2-OG content in plants.

Moreover, the present invention is a plant or seed thereof exhibiting improved growth and/or yield under cultivation conditions where nitrogen is limited to the level below normal cultivation conditions, by introducing a GDH gene or ASPC gene and expressing the gene in a plant body, thereby increasing the 2-OG content. Moreover, the present invention is a method for producing a plant exhibiting improved growth and/or yield under cultivation conditions where nitrogen is limited to the level below normal cultivation conditions, by introducing a GDH gene or ASPC gene and expressing the gene in the plant body, thereby increasing the 2-OG content.

In particular, the present invention is a method for producing a plant exhibiting improved growth and/or yield under cultivation conditions where nitrogen is limited to the level below normal cultivation conditions, in which growth and/or yield equivalent to or greater than growth and/or yield obtained under cultivation conditions with standard application amount (defined for each plant) of nitrogen fertilizer is obtained under cultivation conditions with an amount of nitrogen ranging from between below the lower limit and 1/10-fold the lower limit of the standard application amount of nitrogen fertilizer.

The present invention is a method of cultivating a plant produced by the aforementioned method of the present invention, wherein cultivation is under nitrogen-limited conditions. In particular, the present invention is a method of cultivating a plant developed by the aforementioned method of the present invention under conditions of nitrogen fertilizer application in the range between below the lower limit and 1/10-fold the lower limit of the standard application amount (defined for each plant).

Moreover, the present invention is a method of improving growth and/or yields of plants under cultivation conditions with nitrogen limited to the level below normal cultivation conditions, comprising conducting the foliar application of proline.

### Brief Description of the Drawings

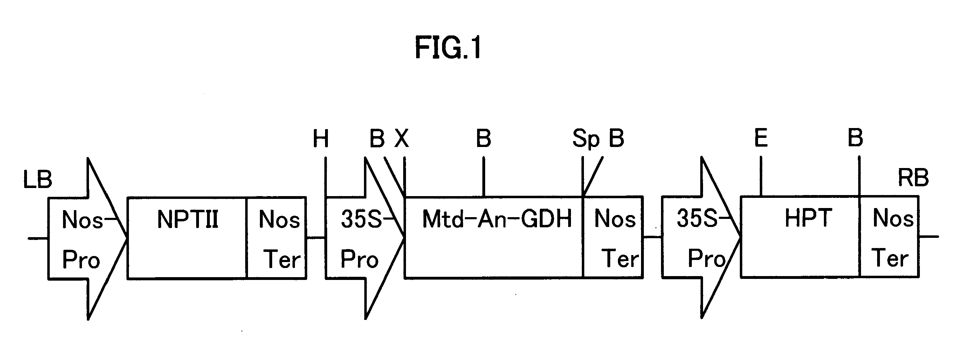

Figure 1 is a schematic diagram of the constructed plasmid vector, in which Nos-Pro is nopaline synthase promoter; NPTII is neomycin phosphotransferase; Nos-Ter is nopaline synthase terminator; 35S-Pro is cauliflower mosaic virus 35S promoter; Mtd-AN-GDH is an *Aspergillus nidulans*-derived glutamate dehydrogenase gene (GDH) with an added mitochondrial transit peptide; HPT is hygromycin phosphotransferase; H is HindIII, B is BamHI, X is Xbal, Sp is Spel, E is EcoRI, LB is left border and RB is right border.

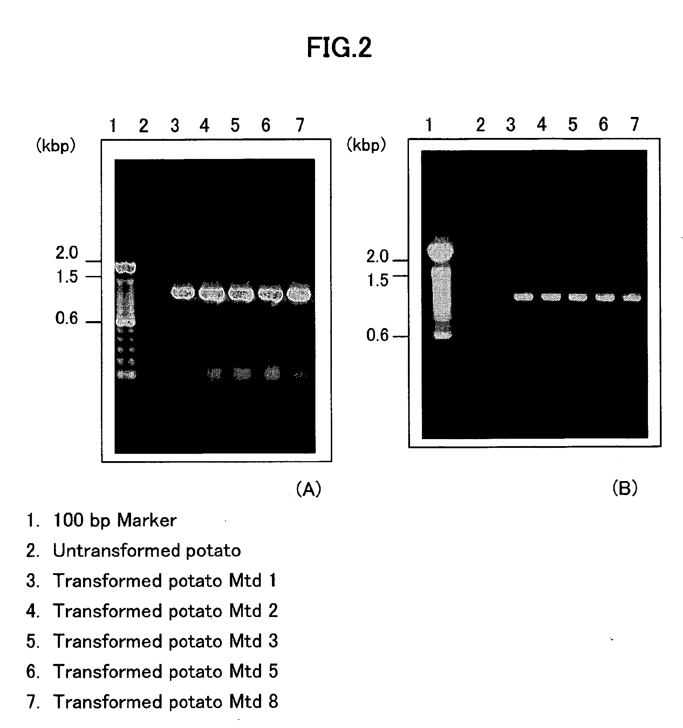

Figure 2 shows the results of PCR analysis of transformed potato, with A indicating the use of An-GDH gene-specific primers and B the use of NPTII gene-specific primers. Lane 1 is a 100 bp marker, lane 2 is untransformed potato, lane 3 is transformed potato Mtd1, lane 4 is transformed potato Mtd 2, lane 5 is transformed potato Mtd3, lane 6 is transformed potato Mtd 5, and lane 7 is transformed potato Mtd 8.

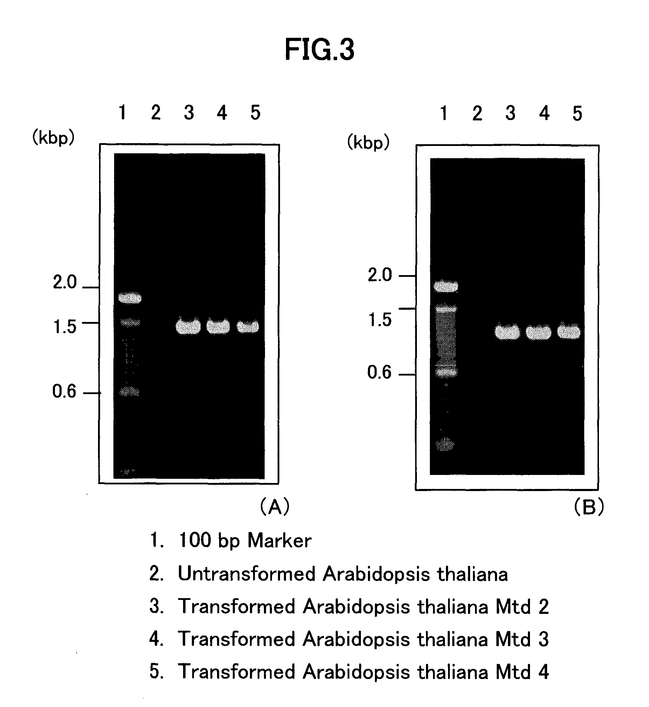

Figure 3 shows the results of PCR analysis of transformed *Arabidopsis thaliana,* with A indicating the use of An-GDH gene-specific primers and B the use of NPTII gene-specific primers. Lane 1 is a 100 bp marker, lane 2 is untransformed *Arabidopsis thaliana*, lane 3 is transformed *Arabidopsis thaliana* Mtd 2, lane 4 is transformed *Arabidopsis thaliana* Mtd 3, and lane 5 is transformed *Arabidopsis thaliana* Mtd 4.

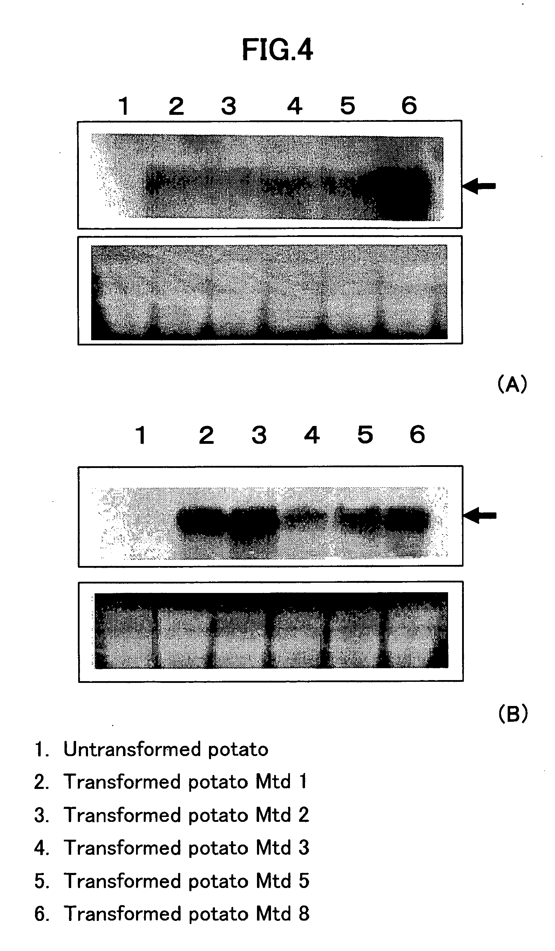

Figure 4 shows the results of Northern analysis of transformed potatoes, with A indicating RNA extracted from leaf tissue and B RNA extracted from tubers. The patterns were obtained by ethidium bromide staining of 10 µg of total RNA that had been electrophoresed (lower patterns in both A and B). The full-length An-GDH gene was used as the probe. Lane 1 is untransformed potato, lane 2 is transformed potato Mtd1, lane 3 is transformed potato Mtd 2, lane 4 is transformed potato Mtd 3, lane 5 is transformed potato Mtd 5 and lane 6 is transformed potato Mtd 8.

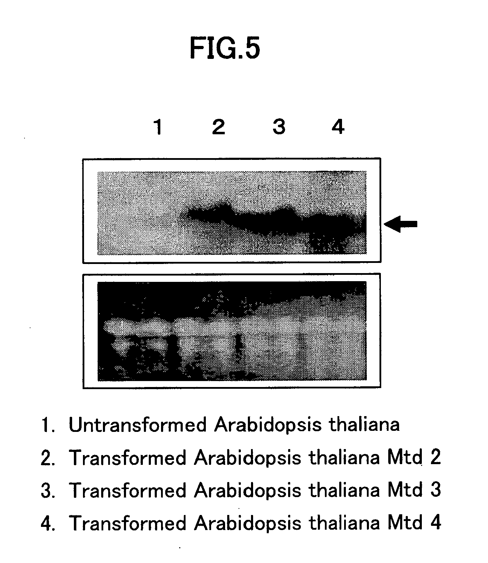

Figure 5 shows the results of Northern analysis of transformed *Arabidopsis thaliana*. RNA extracted from leaf tissue was used. The patterns (lower patterns) were obtained by ethidium bromide staining of 10 µg of total RNA that had been electrophoresed. The full-length An-GDH gene was used as the probe. Lane 1 is untransformed *Arabidopsis thaliana*, lane 2 is transformed *Arabidopsis thaliana* Mtd2, lane 3 is transformed *Arabidopsis thaliana* Mtd 3 and lane 4 is transformed *Arabidopsis thaliana* Mtd 4.

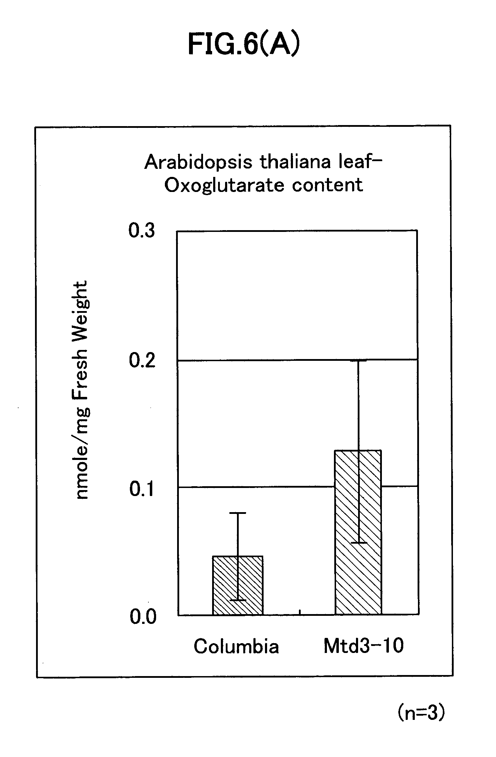

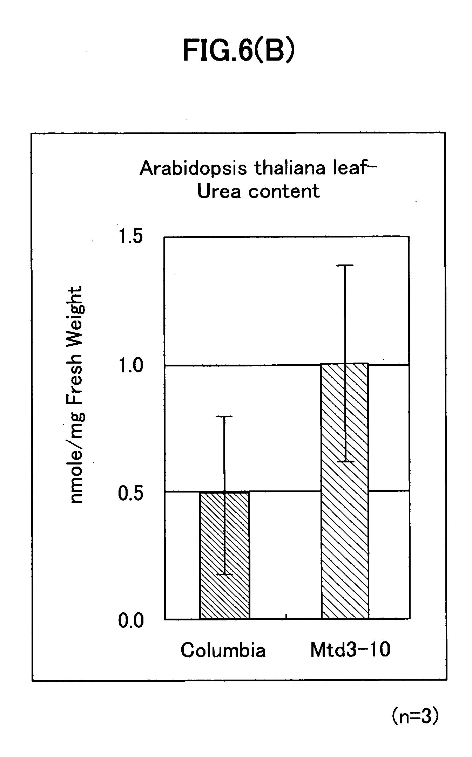

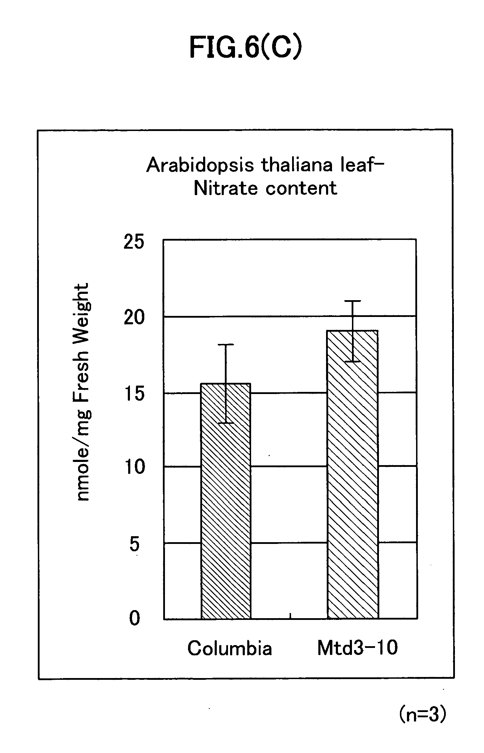

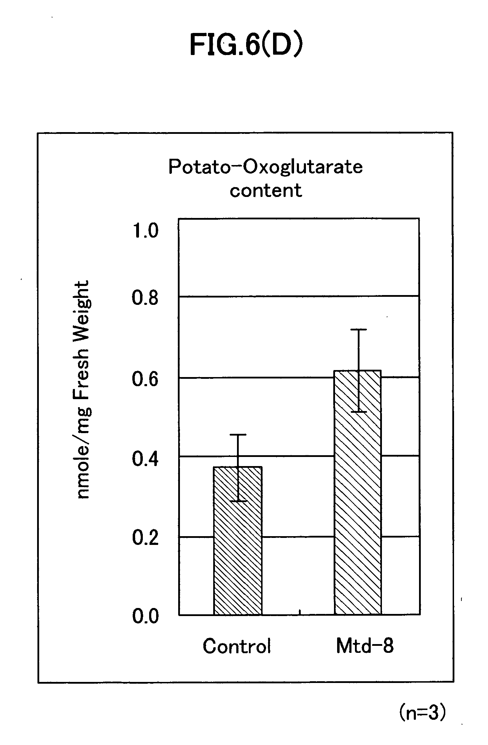

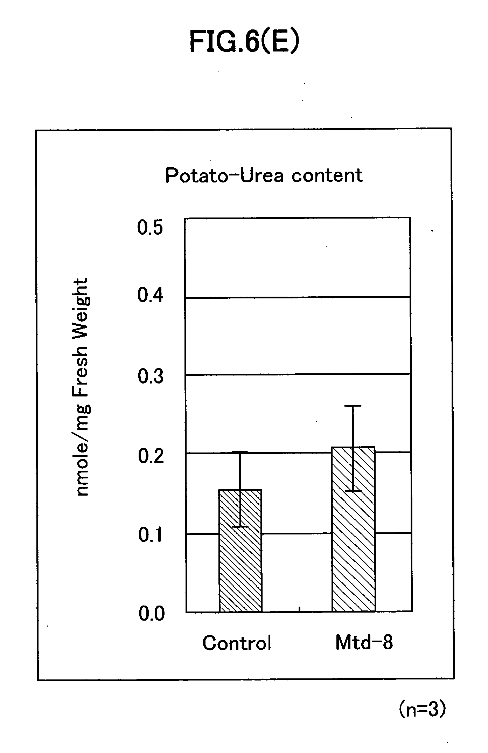

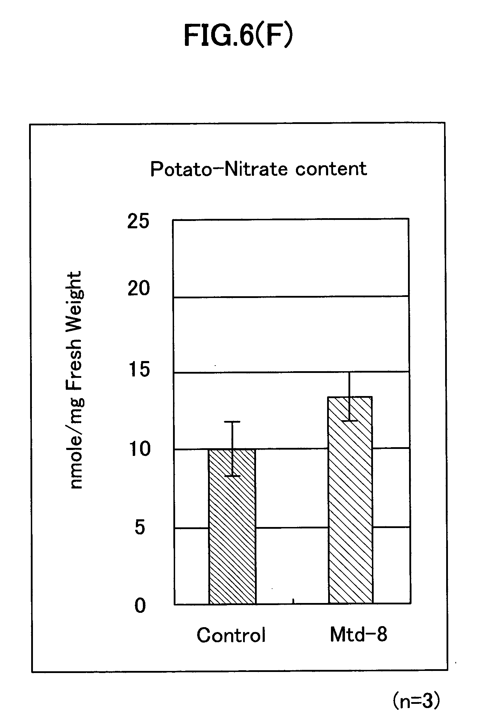

Figures 6A through 6C are graphs showing the 2-oxoglutarate (2-OG), urea and nitrate contents of GDH-transformed plants *(Arabidopsis thaliana*). The vertical axis indicates the mole numbers (nmol) per fresh masses, respectively, with Mtd3-10 representing transformed *Arabidopsis thaliana* (strain no. 3) and Columbia representing untransformed *Arabidopsis thaliana* (n = 3).
Figures 6D through 6F are the graphs showing 2-oxoglutarate (2-OG), urea and nitrate contents of GDH-transformed plants (potato). The vertical axis indicates the mole numbers (nmol) per fresh masses, respectively; with Mtd-8 representing transformed potato and Control representing untransformed potato (n = 3).

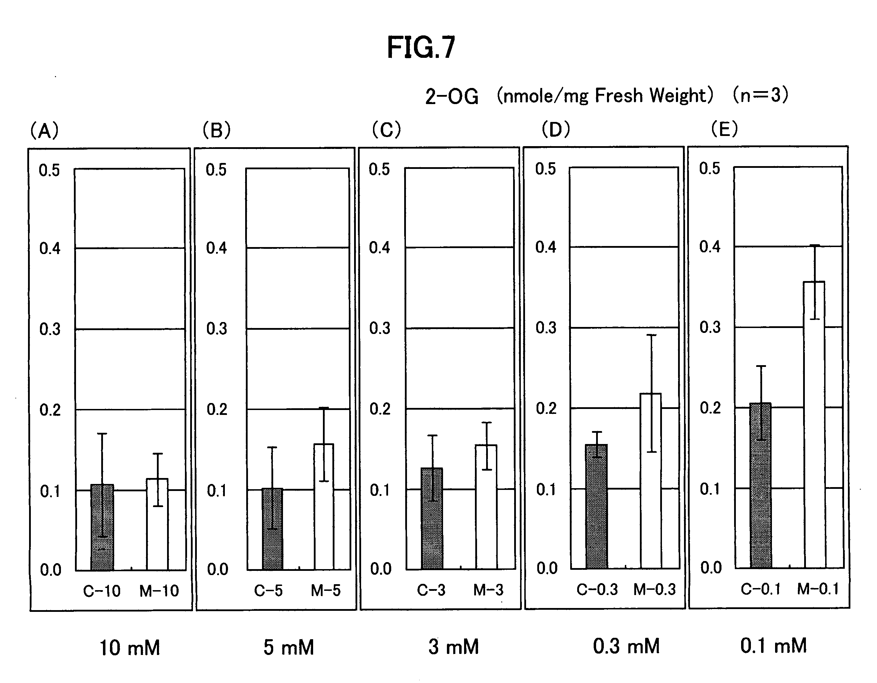

Figure 7 is a graph showing the 2-OG content of *Arabidopsis thaliana* leaf tissue after 3 weeks of cultivation in PNS medium containing different amounts of KNO₃. The mole numbers (nmol) for each live mass are shown on the vertical axis, with C representing untransformed *Arabidopsis thaliana* and M representing transformed *Arabidopsis thaliana* (Mtd 3 strain) (n = 3).

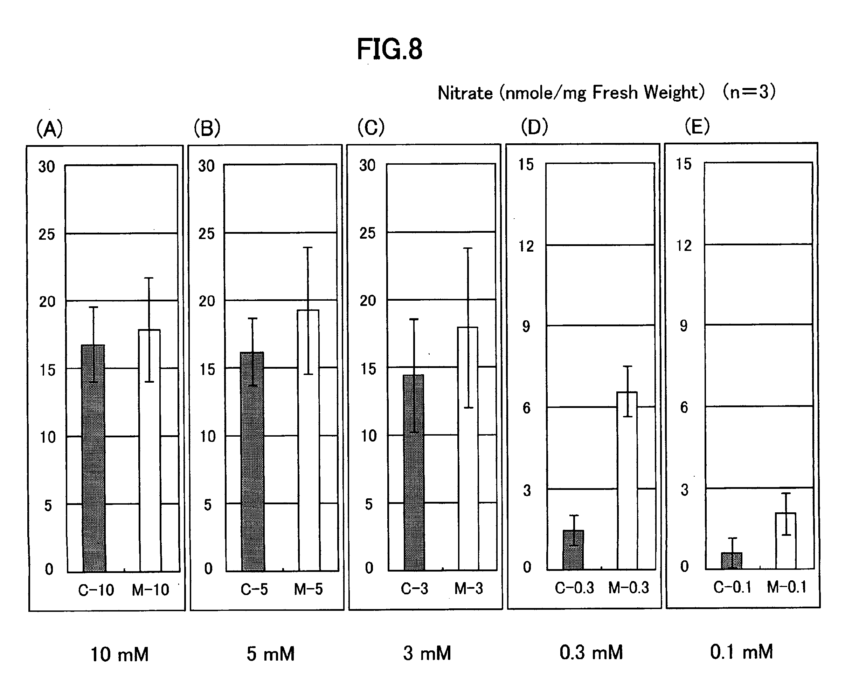

Figure 8 is a graph showing the nitrate content of *Arabidopsis thaliana* leaf tissue after 3 weeks of cultivation in PNS medium containing different amounts of KNO₃. The mole numbers (nmol) for each live mass are shown on the vertical axis, with C representing untransformed *Arabidopsis thaliana* and M representing transformed *Arabidopsis thaliana* (Mtd 3 strain) (n = 3).

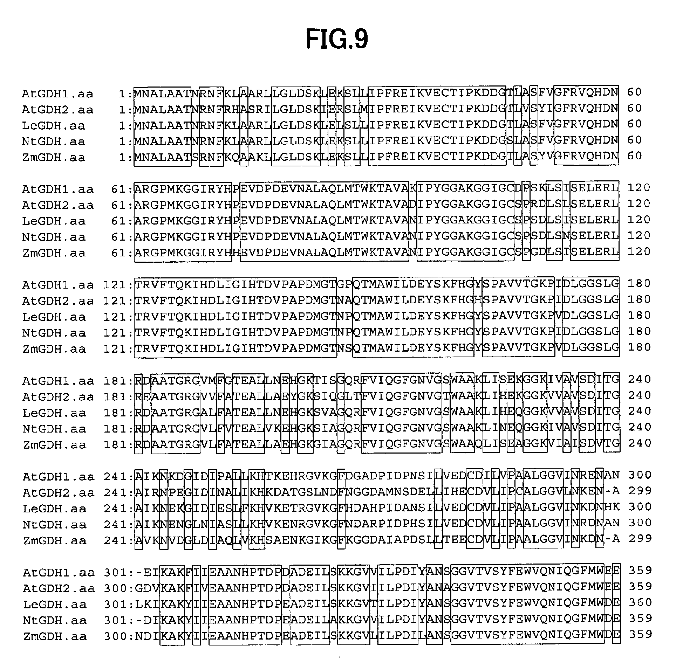

Figure 9 shows alignment results for higher plant glutamate dehydrogenase amino acid sequences, in which AtGDH1 is *Arabidopsis thaliana* GDH1, AtGDH2 is *Arabidopsis thaliana* GDH2, LeGDH is tomato (*Lycopersicon esculentum*) GDH, NtGDH is tobacco (*Nicotiana tabacum*) GDH and ZmGDH is corn (*Zea mays*) GDH.

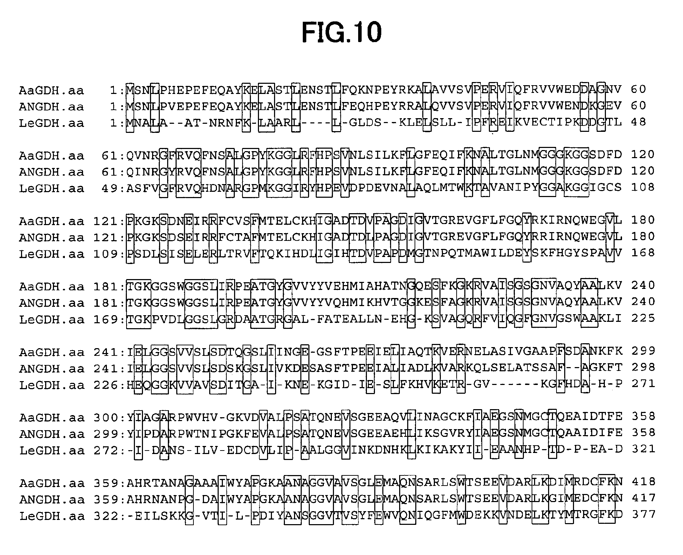

Figure 10 shows alignment results for mold GDH and plant (tomato) GDH amino acid sequences, with AaGDH representing *Aspergillus awamori* GDH, ANGDH representing *Aspergillus nidulans* GDH and LeGDH representing tomato GDH.

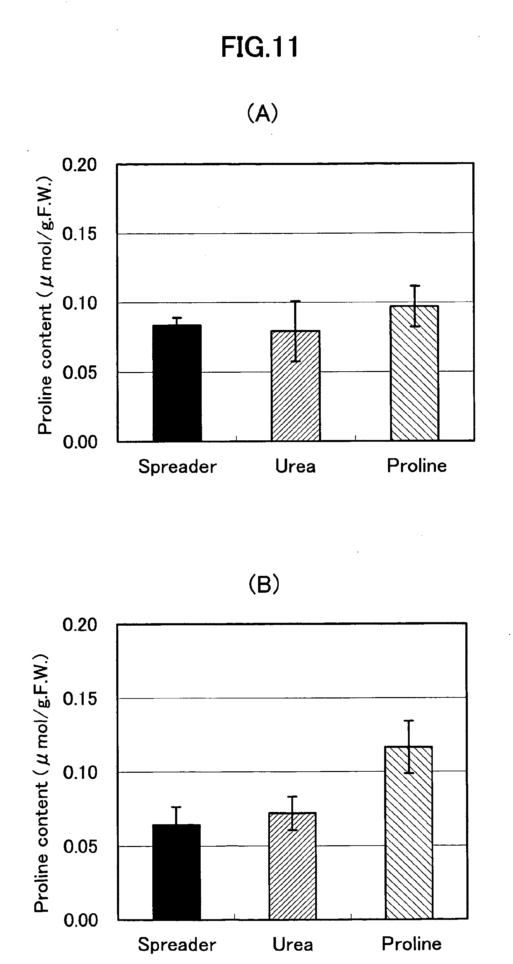

Figure 11 is a graph showing free proline content in leaf tissue 1 and 5 hours after foliar application of an aqueous solution of a spreading agent, urea and proline (n = 6) to potato leaf tissue, respectively, with A indicating 1 hour after application and B indicating 5 hours after application.

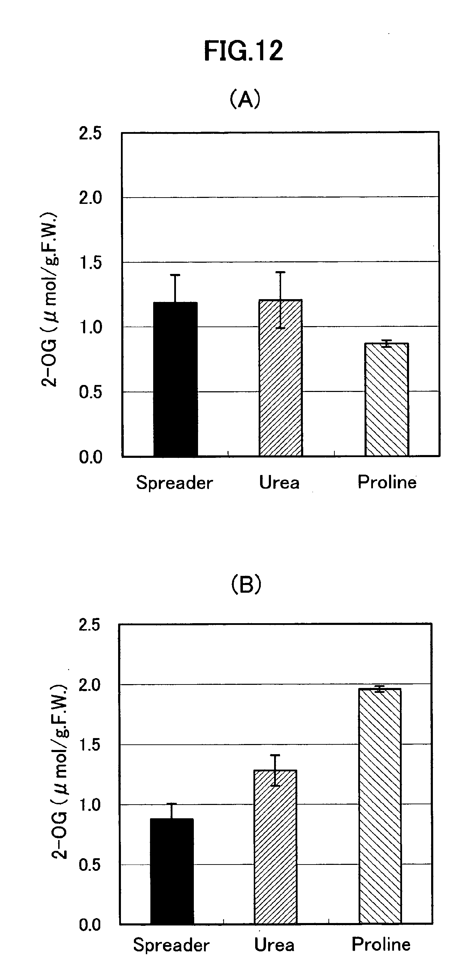

Figure 12 is a graph showing 2-OG content in leaf tissue 1 hour and 24 hours after foliar application of an aqueous solution of a spreader, urea and proline (n = 6) to potato leaf tissue, respectively, with A indicating 1 hour after application and B indicating 24 hours after application.

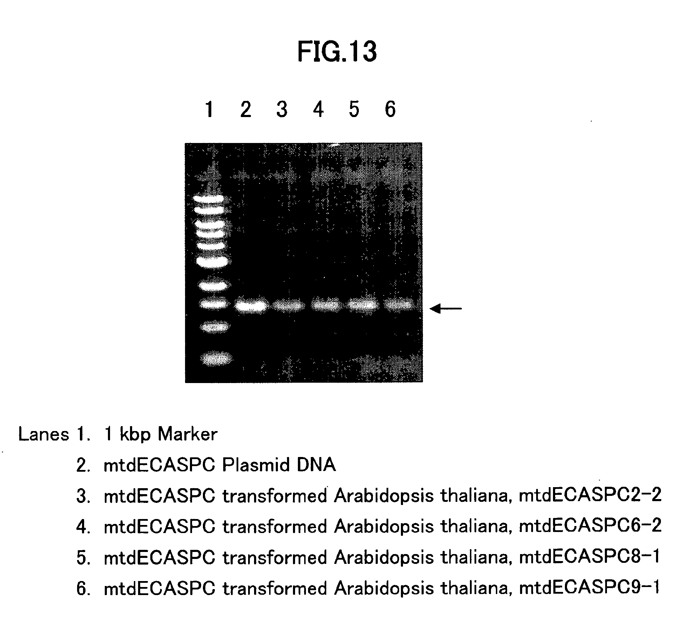

Figure 13 shows genome PCR analysis results for MtdECASPC transformed *Arabidopsis thaliana*. PCR analysis was performed using ECASPC-specific primers, in a reaction of 30 cycles consisting of 1 minute at 94°C, 1 minute at 55°C and 2 minutes at 72°C. Electrophoresis on 1% agarose gel was followed by ethidium bromide staining. Lane 1 is a 1 kbp marker, lane 2 is mtdECASPC plasmid DNA, lane 3 is mtdECASPC transformed *Arabidopsis thaliana* mtdECASPC2-2, lane 4 is mtdECASPC transformed *Arabidopsis thaliana* mtdECASPC6-2, lane 5 is mtdECASPC transformed *Arabidopsis thaliana* mtdECASPC8-1 and lane 6 is mtdECASPC transformed *Arabidopsis thaliana* mtdECASPC9-1.

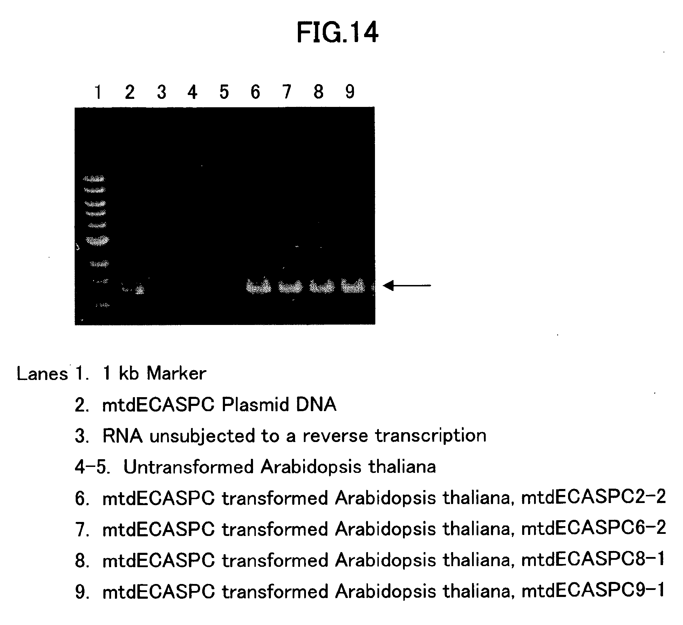

Figure 14 shows RT-PCR analysis of mtdECASPC transformed *Arabidopsis thaliana*. RNA was extracted from *Arabidopsis thaliana* leaf tissue using a Qiagen Plant RNeasy Mini-Kit. RT-PCR was performed using a TaKaRa RNA-PCR Kit with ECASPC-specific primers, in a reaction of 30 cycles consisting of 1 minute at 94°C, 1 minute at 55°C and 2 minutes at 72°C. Electrophoresis on 1% agarose gel was followed by ethidium bromide staining. Lane 1 is a 1 kbp marker, lane 2 is mtdECASPC plasmid DNA, lane 3 is RNA without a reverse transcriptase reaction, lanes 4-5 are untransformed *Arabidopsis thaliana,* lane 6 is mtdECASPC transformed *Arabidopsis thaliana* mtdECASPC2-2, lane 7 is mtdECASPC transformed *Arabidopsis thaliana* mtdECASPC6-2, lane 8 is mtdECASPC transformed *Arabidopsis thaliana* mtdECASPC8-1 and lane 9 is mtdECASPC transformed *Arabidopsis thaliana* mtdECASPC9-1.

### Description of the Preferred Embodiments

As described above, the inventors assumed the existence of a signal molecule that allows plant cells to sense nitrogen nutrient conditions. This is a signal molecule which comprehensively regulates nitrogen absorption and metabolism in plant cells, and it was thought that by artificially increasing and decreasing the amount of this substance it would be possible to maintain nitrogen absorption and metabolism in an enhanced state in plant cells. Moreover, because a plant in such an enhanced state is believed to be capable of actively incorporating nitrogen from exterior, it was thought that nitrogen would be replenished under nitrogen-limited conditions, thus improving growth and yields. Moreover, the inventors supposed 2-oxoglutarate (2-OG) to be this signal molecule.

The first stage of assimilation of inorganic nitrogen into the organic form in plants is mainly incorporation of ammonia into glutamate for producing glutamine, a reaction which is catalyzed by glutamine synthetase (GS) and glutamate synthetase (GOGAT), and as already mentioned above, this GS/GOGAT cycle is thought to be the primary pathway of nitrogen assimilation in plants. Consequently, the degree of nitrogen assimilation or the degree of nitrogen deficiency in plant cells is thought to reflect the amount of 2-OG.

The inventors propose causing over-expression of glutamate dehydrogenase (GDH) activity or aspartate aminotransferase (ASPC) activity as one method of increasing the 2-OG content of plant cells. GDH catalyzes the reversible reactions of incorporating ammonia into 2-OG to produce glutamate and releasing ammonia from glutamate to produce 2-OG. However, it is thought that because in general GDH has a high Km value with respect to ammonia, it is more likely to break down glutamate into 2-OG and ammonia than to incorporate ammonia into 2-OG to produce glutamate, so an increase in 2-OG was expected as a result of the reaction. On the other hand, aspartate aminotransferase is an enzyme that catalyzes a reaction of transposing the amino groups of oxaloacetate, resulting in the production of aspartic acid and 2-OG, so an increase in 2-OG content was expected.

The inventors produced transformed plants into which the GDH gene or ASPC gene is introduced and confirmed actual increase in 2-OG content, and also confirmed that the growth and yields of these plants were improved under cultivation conditions where nitrogen is limited below normal cultivation conditions.

In one embodiment of the present invention, a nucleic acid construct comprising the glutamate dehydrogenase (GDH) gene or aspartate aminotransferase (ASPC) gene is introduced into a plant to obtain a plant which has increased 2-OG content due to expression of the glutamate dehydrogenase (GDH) gene or aspartate aminotransferase (ASPC) gene in the resulting transformed plant, and which has improved growth and/or yield under cultivation conditions where nitrogen is limited below normal cultivation conditions.

In another embodiment of the present invention, the copy numbers and/or transcribed amount of an endogenous GDH gene or endogenous ASPC gene is increased to enhance GDH or ASPC activity, with the 2-OG content of the plant body increased, resulting in a plant with improved growth and/or yield under cultivation conditions where nitrogen is limited below normal cultivation conditions. To increase the transcribed amount, a gene construct capable of expressing a transcription activation factor can be introduced, and/or an enhancer or other cis-functional element can be introduced. Such transcription activation factors and cis-functional elements are well known to those in the field.

There are no particular limits on the origins of the glutamate dehydrogenase used in the present invention, but preferably glutamate dehydrogenase from a microorganism belonging to the genus *Aspergillus* is used. More preferably, glutamate dehydrogenase derived from *Aspergillus nidulans* and *Aspergillus awamori* is used. Moreover, glutamate dehydrogenase having one or more amino acids deleted, substituted or added in the enzyme protein can be used in the present invention as long as it has the aforementioned glutamate dehydrogenase activity and selectively catalyzes a reaction of producing 2-OG and ammonia from glutamate under physiological conditions within plant bodies.

There are also no particular limits on the origins of the aspartate aminotransferase used in the present invention, but preferably aspartate aminotransferase derived from *Escherichia coli* is used. Moreover, aspartate aminotransferase having one or more amino acids deleted, substituted or added in the enzyme protein can be used as long as it has the aforementioned aspartate aminotransferase activity.

Regarding the localization of the glutamate dehydrogenase or aspartate aminotransferase in the cellular organelles, those localized in the mitochondria, chloroplast, peroxisome or the like as well as those localized in the cytoplasm can be used. Consequently, a glutamate dehydrogenase or aspartate aminotransferase gene having added to the N- or C-end thereof either a signal or a transit peptide for transferring the enzyme protein to these cellular organelles can also be used in the present invention.

Such a glutamate dehydrogenase gene or its cDNA can be prepared relatively easily by someone skilled in the art based on published sequence data. In the case of the *Aspergillus nidulans* glutamate dehydrogenase gene, for example, the nucleotide sequence of the genome gene is published under GenBank accession No. X16121. This nucleotide sequence is shown in SEQ ID NO: 1, and the amino acid sequence in SEQ ID NO: 2. Glutamate dehydrogenase cDNA can be obtained easily by synthesizing PCR primers for amplifying DNA fragments so as to include the part encoding the protein based on its sequence, and performing RT-PCR using as the template RNA extracted from cultured cells of *Aspergillus nidulans.* The cDNA sequence of the *Aspergillus nidulans* glutamate dehydrogenase gene is shown in SEQ ID NO: 17. *Aspergillus nidulans* can be obtained for example as ATCC10074 or ATCC11267 from the American Type Culture Collection. The genome sequence for the glutamate dehydrogenase gene of *Aspergillus awamori* is also published under GenBank accession NO: Y15784. This nucleotide sequence is shown in SEQ ID NO: 3, and the amino acid sequence by SEQ ID NO: 4. The cDNA sequence of the *Aspergillus awamori* glutamate dehydrogenase gene is shown in SEQ ID NO: 18. *Aspergillus awamori* itself can be obtained from the American Type Culture Collection as ATCC10548 and ATCC11358. Glutamate dehydrogenase cDNA can be obtained by methods similar to those described above based on these strains and sequence information.

The homology between *Aspergillus nidulans* GDH and *Aspergillus awamori* GDH is about 84% in the amino acid sequence (Figure 10).

In addition, alignment results for the GDH amino acid sequences of *Aspergillus nidulans, Aspergillus awamori* and tomato (*Lycopersicon esculentum*) are shown in Figure 10, while alignment results for the amino acid sequences of *Arabidopsis thaliana* GDH1, *Arabidopsis thaliana* GDH2, tomato GDH, tobacco (*Nicotiana tabacum*) GDH and corn (*Zea mays*) GDH are shown in Figure 9.

In the present invention, plants which have improved growth and/or yield under cultivation conditions where nitrogen is limited below ordinary cultivation conditions and which have increased 2-OG content of the plant can be obtained using genes which encode a variety of enzymes which produce 2-OG from glutamate and which are different from the glutamate dehydrogenase gene. Examples of such enzymes include aspartate aminotransferase and alanine aminotransferase.

For example, the nucleotide sequence of genome DNA for the aspartate aminotransferase gene of *E. coli* strain K-12 is recorded under GenBank accession No. X03629. The aspartate aminotransferase gene can be easily obtained by synthesizing PCR primers based on that sequence so as to amplify a DNA fragment comprising the region encoding the protein, and performing PCR using these primers with chromosome DNA extracted from *E. coli* strain K-12 as the template.

In the present invention, the growth and/or yields of plants under nitrogen-limited cultivation condition where nitrogen is limited below normal cultivation conditions can be improved by applying proline to leaf surfaces as a means of increasing 2-OG content in plants. The proline applied to the leaves will be incorporated into the leaf tissue and metabolized into glutamate. Moreover, because this glutamate is metabolized into 2-OG by endogenous glutamate dehydrogenase, it can increase the 2-OG content in the tissue. A plant the 2-OG content of which has been increased in this way by application of proline to the leaf surfaces has improved growth and/or yield under conditions in which the amount of available nitrogen during the cultivation period is limited below ordinary cultivation conditions.

A nucleic acid construct that can be used in the present invention can be prepared using ordinary methods known to those skilled in the art. For example, Sambrook et al, Molecular Cloning―Laboratory Manual, 2^{nd} Ed., Cold Spring Harbor Laboratory Press or the like can be consulted with regards to molecular biology techniques including methods for isolating the nucleic acid construct and determining its sequence. Alternatively, in some cases genetic amplification such as PCR is necessary for preparing a nucleic acid construct which can be used in the present invention, and F. M. Ausubel et al (eds.), Current Protocols in Molecular Biology, John Wiley & Sons, Inc. (1994) can be consulted for such methods.

Nucleic acid constructs which can be used in the present invention may normally include suitable promoters which function in plant cells, such as the nopaline synthase gene or cauliflower mosaic virus 35S promoter (CaMV35S), suitable terminators such as the nopaline synthase gene terminator, other sequences which are necessary or useful for expression, and marker genes for selecting transformants, such as drug resistance genes for kanamycin resistance, G418 resistance, hygromycin resistance and the like for example.

Promoters that can be used for such constructs may be constitutive promoters or organ-specific or growth stage-specific promoters, and may be selected depending on the host, the necessary amount of expression, the organs in which expression is particularly desired or the growth stage. A preferred embodiment of the present invention uses a powerful promoter that is expressed in a way non-specific to organs and growth stages, and one example of such a promoter is the CaMV35S promoter. Organ-specific promoters that can be used include the phaseolin gene promoter and the patatin gene promoter. The most desirable embodiment of the present invention uses a construct that drives the glutamate dehydrogenase gene or aspartate aminotransferase gene by means of a strong structural promoter such as the CaMV35S promoter.

There are no particular limits on the gene introduction method used in the present invention, and a method known to those skilled in the art for introducing genes into plant cells or plant bodies can be selected according to the host. For example, one embodiment of the present invention employs a gene introduction method using *Agrobacterium.* A binary vector is preferably used in this transforming system. When using *Agrobacterium,* the nucleic acid construct used in gene transformation may also include a T-DNA region flanking the DNA sequence which is to be introduced into the plant cells. In a preferred embodiment, the introduced sequence is inserted between left and right T-DNA border sequences. The suitable design and construction of a transformation vector based on such T-DNA is well known to those skilled in the art. The conditions for infecting a plant with *Agrobacterium* having such a nucleic acid construct are also well known to those skilled in the art. Regarding such techniques and conditions, see Shujunsha Pub., Saibou Kougaku Supplement, "Experimental Protocols in Model Plants: Rice and *Arabidopsis thaliana*" (1996).

Another gene introduction method can be used in the present invention. Examples of gene introduction methods which can be used include DNA protoplast introduction using polyethylene glycol or calcium, protoplast transformation using electroporation, and introduction using a particle gun and the like.

There are no particular limits on the plant that is genetically manipulated in this way, and it is desirable to use a species which is easy to transform and for which a regenerative system has been established, except for the cases where the transformation is performed using the whole plant. In addition to the aforementioned properties, a suitable plant in the present invention is one for which mass cultivation techniques have been established so as to reduce the environmental burden of agriculture. Examples of plants which are suited to the present invention include all plants in the Brassica family as well as tomatoes, potatoes, corn, wheat, rice, sugar cane, soy beans, sorghum and the like. Examples of plants that are difficult to supply with large amounts of nitrogen include trees and fruit trees, such as poplar, eucalyptus and apple trees. The activities and properties of GDHs and ASPCs from these plants are equivalent to those of *Aspergillus nidulans*-derived GDH (SEQ ID Nos. 1 and 2) and *E. coli* K-12 strain-derived ASPC (SEQ ID Nos. 19 and 20) used in one embodiment of the present invention. Consequently, 2-OG content in these plants can by increased by introducing the *Aspergillus nidulans*-derived GDH gene (SEQ ID NO: 1) or *E. coli*-derived ASPC gene (SEQ ID NO: 19). A GDH gene or ASPC gene derived from any of these plants could also be used in the same way in the present invention. There are no particular limits on what organs or cells are subjected to genetic manipulation as described above, and they can be selected depending on the host, the genetic introduction method and the like. Examples include organ explant, pollen, cultured cells, germ, plant bodies and the like, but are not limited to these.

Plant cells or the like which have been manipulated as described above are selected for transformation. Selection can be based on expression of a marker gene which is present on the nucleic acid construct used for transformation. For example, when the marker gene is a drug resistance gene, the manipulated plant cells or the like can be cultured or grown on a medium containing a suitable concentration of an antibiotic, herbicide or the like. If the marker gene is a beta-glucuronidase gene, luciferase gene or the like, the transformant can be selected by screening for its activity. When the transformant identified in this way is not a plant body but a protoplast, callus, explant or the like, it can be regenerated into a whole plant body. A method known to someone with skill in the art can be used for regeneration depending on the host plant.

The resulting whole plant can be cultivated by ordinary methods or in other words under the same conditions as a non-transformant, and a variety of molecular biology techniques can be used in addition to the aforementioned selection based on marker genes to identify transformed plants comprising the nucleic acid construct of the present invention. For example, Southern hybridization or PCR can be used to detect the presence or absence and the structure of recombinant DNA-inserted fragments. Northern hybridization, RT-PCR or the like can be used to detect and measure RNA transcription products derived from the introduced nucleic acid construct.

Next, expression of the glutamate dehydrogenase gene or aspartate aminotransferase gene by the resulting transformant is evaluated by measuring the amount of protein, mRNA or enzyme activity for the glutamate dehydrogenase or aspartate aminotransferase. For example, the amount of the glutamate dehydrogenase protein or aspartate aminotransferase protein can be evaluated by Western blotting or the like, and the amount of mRNA by Northern blotting or quantitative RT-PCR. Glutamate dehydrogenase activity in plant extract can be measured by the methods of Ameziane et al (Ameziane, R., Bernhard, K. and Lightfood, D., *Plant and Soil* 221: 47-57, 2000). Aspartate aminotransferase activity in plant extract can be measured by the methods of Chao et al (Chao, Y. P., Lai, Z.I., Chen, P. and Chen, J.T., *Biotechnology Progress* 15:453-458, 1999).

A transformed plant in which expression or increased expression of the glutamate dehydrogenase gene or aspartate aminotransferase gene has been confirmed is further evaluated for 2-OG content. 2-OG content can be assayed for example by the enzyme method using a plant extract prepared by crushing all or a portion of the transformed plant body. The plant extract can be prepared and 2-OG assay by the methods of Usuda (Usuda, H., *Plant Physiol.* 78:859-864, 1985) for example.

If the 2-OG content of the above-ground parts (for example, the leaves, stems or both, the floral organs or part of the fruit) or the below-ground parts (for example, the roots or tubers) under normal cultivation conditions and under conditions where nitrogen is limited to the level below normal cultivation conditions is increase 1.2-fold or more as compared with the original line into which the gene has been introduced, it is considered that the 2-OG content is increased.

A transformed plant in which increased 2-OG content has been confirmed in this way can be cultivated under cultivation conditions where nitrogen is limited below normal cultivation conditions during the cultivation period, and its growth or yield can be evaluated. "Conditions where nitrogen is limited below normal cultivation conditions" as used herein refers to a condition supplying an amount of nitrogen which is less (for example, such as 70% or less, or preferably 50% or less) than the standard amount of nitrogen, or in other words the standard nitrogen application rate, which is normally used in cultivating each plant. In particular, according to the method of the present invention, growth and/or yield equivalent to or greater than that achieved under cultivation conditions with application of the standard amount of nitrogen is achieved under cultivation conditions in which an amount of nitrogen raging between below the lower limit of the standard amount and 1/10-fold the lower limit is provided. For example, standard amounts of nitrogen application are established for individual crops. In the case of potatoes for example, the standard amount of nitrogen is 7 kg per 10 a (JRT Japanese Society of Root and Tuber Crops home page: http://www.jrt.gr.jp/minilpm_index.html; Minoru Yoshida, Enjoy Learning All About Potatoes, Nobunkyo, 1988), while it is 5 to 15 kg per 10 a for rice (A Manual of Experiments for Plant Biology, Ed. Kokichi Hinata and Teruyoshi Hashiba, Soft Science Publications, 1995; Nara Agricultural Technology Center home page, http://www.naranougi.jp/sehi-kijyun/sehikijyun-index.html). In *Arabidopsis thaliana* and other experimental plants, medium comprising 5 mM or more nitrate is considered standard medium suitable for growth and development (Martinez-Zapater, J.M. and Salinas, J. Ed., Methods in Molecular Biology, *Arabidopsis thaliana* Protocols, Humana Press, New Jersey, 1998). Such a standard nitrogen application rate or standard nitrogen amount in medium is well known to someone with skill in the art.

The transformed plant is cultivated under conditions with nitrogen limited below normal cultivation conditions or in other words, as described above, with an amount of nitrogen in the range between below the lower limit of the standard nitrogen application rate and 1/10 the lower limit, and its growth and/or yield is evaluated. Growth can be evaluated by measuring the above-ground parts or the plant length, number of leaves, or fresh weight or dry weight of the plant as a whole. Improved growth means that at least one of the parameters regarding a whole plant body or a part of a plant body (such as the plant length, number of leaves, fresh weight or dry weight) is 1.2-fold or more as compared with that of a control plant cultivated around the same time under the standard nitrogen application rate. Yield can be evaluated based on the harvested part of the plant, such as the number or individual weight of the tubers for potato or of the seeds for rice, or the total weight of the harvested parts. Consequently, improved (or increased) yield means that this harvested part has 1.2-fold or more as compared with that of a control plant which is cultivated around the same time under the standard nitrogen application rate.

Once a transformed plant which has better growth and yield than a untransformed plant used as a control, under conditions in which nitrogen use is limited below normal cultivation conditions has been identified, the genetic stability of its characteristics is then investigated. To this end, the plant can be raised, cultivated and the seeds are collected under normal conditions, and characteristics and their segregation are analyzed in the progeny. The presence or absence, location, expression and the like of the introduced nucleic acid construct in the progeny can be analyzed in the same way as for the first-generation (T1 generation) transformant.

In a transformed plant the growth and yield of which are increased under conditions with nitrogen limited below normal cultivation conditions, or in other words a transformed plant the growth of which is no longer inhibited under nitrogen-limited conditions, a sequence derived from a nucleic acid construct incorporated into the introduced genome may be either hemizygous or homozygous, and either a hemizygote or homozygote can be derived in the progeny by cross-breeding or the like as necessary. The sequence derived from the nucleic acid construct integrated into the genome is isolated in the progeny by Mendelian genetics. Consequently, a homozygous plant is preferably used for obtaining descendent plants with stable characteristics.

In most cases, the transformant has an exogenous gene inserted in only one genetic locus, but multi-copy transformants with insertions in multiple gene loci are not unusual. A single-copy transformant is preferred in the present invention for purposes of stability and the like of the introduced gene. For example, a single-copy transformant with the introduced gene in a single gene locus can be selected by investigating the segregation ratio of kanamycin resistance in T2 (the second generation). If T1 is hemizygous and the introduced gene is in a single gene locus, kanamycin resistance and sensitivity will be segregated 3:1 in T2 according to Mendel's law. When there are multiple copies of the introduced gene, the frequency of the resistant transformant increases. Consequently, a single-copy transformant can be obtained by plating the resulting T2 seeds in medium containing kanamycin, selecting a line which exhibits a 3:1 segregation ratio, and selecting those transformants in which the introduced gene is thought to be present in a single gene locus.

The resulting transformed plant can be cultivated under the same conditions as a naturally-occurring plant of the same species, and, when cultivated under conditions where the nitrogen supply is limited to the level below normal cultivation conditions, can provide a harvest equal to or greater than that obtained from a untransformed plant. Moreover, seeds can be obtained from a transformed plant prepared in this way. Seeds can be easily obtained by the same methods used for an untransformed plant of the same species. If necessary, the resulting seeds can be stored, sterilized, and rid of harmful insects by normal methods known to those with skill in the art.

The 2-OG content of a plant can thus be increased by gene introduction, and the 2-OG content of the plant can also be further increased by applying the amino acid proline to the leaves. Specifically, an aqueous solution of 20 mg/1 to 500 mg/l of proline can be sprayed or otherwise applied together with a suitable spreader to the leaf surfaces of the plant. The 2-OG content of a plant which has received such foliar application of proline can be evaluated by methods similar to those described above. Moreover, the growth effects under nitrogen-limited conditions can also be evaluated under conditions and by methods similar to those described above.

The present invention is explained more specifically and in more detail below by means of the following examples involving the preparation and yield surveys and component analysis of plants manipulated for overexpression of the NADP-GDH gene or aspartate aminotransferase gene, and by the following examples involving yield surveys and component analysis of plants which had received foliar application of proline.

### Examples

### Example 1. Isolation of Aspergillus nidulans-derived NADP-dependent GDH gene, and construction of Ti plasmid vector

### (1) Isolation of Aspergillus nidulans-derived NADP-dependent GDH gene

*A. nidulans* was seeded on potato dextrose agar medium and cultured overnight at 30°C, and the resulting colony was cultured for 2 days in liquid dextrose medium. Total RNA was prepared from the proliferated cells.

mRNA was purified using a Poly(A) Quick mRNA Isolation Kit (Stratagene), and First-strand cDNA was prepared using a First-strand cDNA Synthesis Kit (Amersham Bioscience). A PCR reaction was performed using the prepared First-strand cDNA as the template. The PCR reaction conditions were 35 cycles of 3 minutes at 94°C, 45 seconds at 94°C, 30 seconds at 59°C and 90 seconds at 72°C, followed by 10 minutes at 72°C, using a Perkin-Elmer PCR system 2400. The primers used were 5'-TCT AGA ATG TCT AAC CCC CTT GTT GAG-3' (SEQ ID NO: 5) and 5'-GAG CTC TCA CCA CCA GTC ACC CTG GTC-3' (SEQ ID NO: 6). A roughly 1.4 kbp band was confirmed, matching the size of the expected gene. The resulting PCR product was cloned using a TA Cloning Kit (Invitrogen). The nucleotide sequence of the resulting clone was determined with a sequencer (ABI 377A). This matched the known *A. nidulans*-derived NADP-dependent GDH gene, and was given as An-GDH (SEQ ID NO: 17).

### (2) Construction of Ti plasmid vector

A nucleotide sequence encoding a mitochondrial transit peptide was added to the resulting gene. This was a 5'-end sequence from the tomato-derived NAD-dependent GDH gene which was obtained by PCR using the primers 5'-CTG CAG ATG AAT GCT TTA GCA GCA AC-3' (SEQ ID NO: 7) and 5'-TCT AGA TAA ACC AAG AAG CCT AGC TG-3' (SEQ ID NO: 8). Ligation of the resulting An-GDH gene with the nucleotide sequence encoding the mitochondrial transit peptide was accomplished using the four primers 5'-TCT AGA ATG AAT GCT TTA GCA GCA AC-3' (SEQ ID NO: 9), 5'-GGG AAG GTT TAG ACA TTA AAC CAA GAA GCC T-3' (SEQ ID NO: 10), 5'-AGG CTT CTT GGT TTA ATG TCT AAC CTT CCC-3' (SEQ ID NO: 11) and 5'-GAG CTC TTA CGC CTC CCA TCC TCG AA-3' (SEQ ID NO: 12). The resulting GDH gene with added mitochondrial transit peptide sequence was designated as Mtd-An-GDH. This Mtd-An-GDH gene was substituted for the GUS region of the Ti plasmid pIG121-Hm, an *Agrobacterium* transforming vector. The resulting Ti plasmid was introduced into *Agrobacterium tumefaciens* EHA101, and used in transforming *Arabidopsis thaliana* and potato plants.

### Example 2. Development of potato transformant

Potatoes (May Queen cultivar) were transformed according to the methods of Gordon et al (Ref: *Plant Cell Reports*, 1993, 12:324-327). Sterilely-induced microtubers were cut, and tuber discs were prepared, placed on MS agar medium supplemented with 2 mg/l zeatin and 0.1 mg/l indole, and cultured for 24 hours at 25°C with a 16 hour day length. *Agrobacterium* comprising the constructed gene was inoculated on YEP medium (10 g/l Bacto Tryptone, 10 g/l yeast extract, 1 g/l glucose) containing 50 mg/l kanamycin and 50 mg/l hygromycin, and shaking cultured overnight at 28°C. The *Agrobacterium* culture was added to infect the tuber discs that had been cultured for 24 hours. After 10 minutes, the excess *Agrobacterium* was removed using sterilized filter paper, transferred to an aforementioned dish, and cultured for 24 hours under the same conditions. The tuber discs were then transferred on MS agar medium containing 50 mg/l kanamycin, 300 mg/l cefotaxime hydrochloride, 2 mg/l zeatin and 0.1 mg/l indoleacetic acid, and the transformants were selected. The regenerated shoots were transferred once again to the aforementioned selection medium, and resistance was confirmed. Shoots that clearly exhibited kanamycin resistance were transplanted to MS agar rooting medium containing 50 mg/l kanamycin and 300 mg/l cefotaxime hydrochloride to induce root regeneration. Shoot apices were cut at least three times from the rooted plant and transplanted to selection rooting medium, kanamycin resistance was confirmed, and chimeras were excluded. The resulting 5 individuals were acclimatized to soil to obtain tubers.

### Example 3. Production of Arabidopsis thaliana transformants

Gene introduction into *Arabidopsis thaliana* was accomplished according to the methods of Bechtold et al (Ref: C. R. Acad. Sci. Paris, *Life Science* 316:1194-1199, 1993). *Arabidopsis thaliana* seeds were planted on culture soil, and after 10 days of cultivation at 24°C with a 16 hour day length, the seedlings were transplanted one by one to individual pieces of rock wool, and cultivated for a further two weeks under the same conditions. The plants were pinched as soon as they began to bolt, and were then cultivated for another week. *Agrobacterium* was shaking cultured for 24 hours at 28°C in YEP culture containing 50 mg/l kanamycin and 50 mg/l hygromycin, and cells were collected by centrifugation (7,000 rpm, 10 minutes). The cells Were suspended in suspension medium for infiltration (1/2 MS salts, 1/2 B5 vitamin, 5% sucrose, 0.5 g/i MES, 0.044 µM benzylaminopurine, pH 5.7). After removal of flowering organs which had already flowered or set fruit, the plants were immersed in the *Agrobacterium* suspension liquid, and treated in a desiccator under reduced pressure (40 mmHg) for 15 minutes. The treated plants were cultivated for 1 month, and seeds were collected. The seeds were planted on MS agar medium containing 50 mg/l kanamycin and 100 mg/l cefotaxime, and selected. Three of the resulting transformants were further cultivated to 3^{rd} generation (T3), and the lines that no longer exhibited segregation of kanamycin resistance were used in analysis.

### Example 4. Confirmation of the introduced gene

DNA was extracted from selected individuals exhibiting kanamycin resistance (5 potato and 3 *Arabidopsis thaliana* plants) and from non-infected plants. DNA was extracted according to the methods of Honda et al (Ref: Honda and Hirai, 1990 *Jpn J Breed* 40: 339-348). PCR analysis was performed using the extracted DNA, the An-GDH gene-specific primers 5'-TCT AGA ATG TCT AAC CCC CTT GTT GAG-3' (SEQ ID *NO:* 13) and 5'-GAG CTC TCA CCA CCA GTC ACC CTG GTC-3' (SEQ ID NO: 14) and the primers 5'-CCC CTC GGT ATC CAA TTA GAG-3' (SEQ ID NO: 15) and 5'-CGG GGG GTG GGC GAA GAA CTC CAG-3' (SEQ ID NO: 16) for amplifying the NPTII gene in the vector. The reaction consisted of 3 minutes at 94°C followed by 35 cycles of 45 seconds at 94°C, 30 seconds at 55°C and 90 seconds at 72°C followed by 10 minutes at 72°C, using a Perkin-Elmer PCR System 2400. The PCR product was electrophoresed on 1% agarose gel, and stained with ethidium bromide.

As a result, an An-GDH gene-specific band (about 1.5 kbp) and an NPTII gene-specific band (about 1.1 kbp) were confirmed in the selected transformed potatoes (Figure 2) and transformed *Arabidopsis thaliana* (Figure 3), while no bands were confirmed in the non-infected plants, indicating that a T-DHA region comprising the An-GDH gene had been introduced into the transformed potatoes and transformed *Arabidopsis thaliana*.

### Example 5. Confirmation of introduced gene expression (Northern analysis)

Expression of the introduced gene was confirmed by Northern analysis using transformed potatoes and transformed *Arabidopsis thaliana* in which introduction of the An-GDH gene had been confirmed.

RNA was extracted by the SDS-phenol method from transformed potato leaf tissue and tubers and transformed *Arabidopsis thaliana* leaf tissue. The extracted RNA was electrophoresed on 1.2% agarose gel containing 18% formaldehyde, and stained with ethidium bromide. After being blotted on a nylon membrane (HybondN⁺), it was fixed with UV and hybridized. The full length of the An-GDH gene was used as the probe. The Northern blot and probe were prepared using a Roche Diagnostics DIG-High Prime DNA Labeling and Detection Starter Kit II and PCR DIG Probe Synthesis Kit.

As a result, an An-GDH gene-specific band was confirmed in the leaf tissue and tubers of the transformed potato plants (Figure 4), showing that the introduced gene was being transcribed and expressed in the leaf tissue and in the tubers of the transformed potato plants. An An-GDH gene-specific band was also confirmed in the leaf tissue of the transformed *Arabidopsis thaliana* (Figure 5), indicating that the introduced gene was being transcribed and expressed in the transformed *Arabidopsis thaliana* leaf tissue. These An-GDH-specific bands were not observed in the untransformed potato or *Arabidopsis thaliana* plants.

### Example 6. Expression of introduced gene (NADP-GDH activity)

NADP-GDH activity was measured to confirm expression of the introduced gene using transformed potato and *Arabidopsis thaliana* plants in which introduction of the An-GDH gene had been confirmed.

Activity was measured according to the methods of Ameziane et al (Ref: *Plant and Soil,* 2000, 221:47-57). The transformed potato (about 0.2 g) and transformed *Arabidopsis thaliana* leaf tissue (about 0.2 g) were freeze dried with liquid nitrogen and crushed in a mortar, and 5x by weight of extraction buffer (200 mM Tris (pH 8.0), 14 mM beta-mercaptoethanol, 10 mM L-cysteine-HCl, 0.5 mM PMSF) was added. This was transferred to a centrifugation tube and centrifuged for 30 minutes at 4°C, 12,000 rpm, and the supernatant was ultrafiltered (Millipore, Ultrafree 0.5 filter unit, Biomax-10) and washed 3 times in extraction buffer.

Activity was measured by measuring the reduction in absorbance at a wavelength of 340 nm when a reaction mixture comprising 100 mM Tris (pH 8.5), 20 mM 2-oxoglutaric acid, 10 mM CaCl₂, 0.2 mM NADPH and 200 mM NH₄Cl was added to the previously extracted raw enzyme solution. The protein concentration in the extracted crude enzyme solution was measured by the Bradford method using bovine serum albumin (BSA) as the standard protein.

As a result, the transformed potato exhibited about 20 to 50 times the activity of the untransformed potato (Table 1). The transformed *Arabidopsis thaliana* also exhibited about 5 to 12 times the activity of the untransformed *Arabidopsis thaliana* (Table 2). Since the non-transformants exhibited almost no NADP-GDH activity, the activity was attributed to expression of the introduced An-GDH gene.

**Table 1. NADP-GDH activity in transformed potatoes and untransformed potato**

| | NADP-GDH activity (µmol/min/mg protein) |
|---|---|
| Non-transformant | 0.11 |
| Transforman | |
| Mtd 2 | 2.12 |
| Mtd 5 | 4.64 |
| Mtd 8 | 5.85 |

**Table 2. NADP-GDH activity in transformed Arabidopsis thaliana and untransformed Arabidopsis thaliana**

| | NADP-GDH activity (nmol/min/mg protein) |
|---|---|
| Non-transformant | 30.0 ± 5.2 |
| Transformants | |
| Mtd 2 | 393.5 ± 71.3 |
| Mtd 3 | 356.5 ± 70.3 |
| Mtd 4 | 153.9 ± 29.5 |

### Example 7. 2-oxoglutarate (2-OG), urea and nitrate content of GDH gene-introduced Arabidopsis thaliana and potato

The 2-OG, urea and nitrate contents of leaf tissues from transformed potato (Mtd 8) and transformed *Arabidopsis thaliana* (Mtd) were measured. For the potato, leaf tissue of a vigorously growing plant body that had been cultivated for 1 month in a mixed soil of 500 g Power Soil and 500 g vermiculite was used as the material. The leaf tissue of a plant that had been grown for 3 weeks in PNS medium (containing 5 mM KNO₃) was used for the *Arabidopsis thaliana.* Extraction was according to the methods of Agarie et al (Ref: *Plant Science* 2002, 162:257-265). The leaf tissue (about 0.1 g) was first crushed using liquid nitrogen, and then mixed well after addition of 200 µl of 3% HCO₄. An additional 200 µl of 3% HCO₄ was added to the remaining precipitant, and mixed well. After 10 minutes of centrifugation at 12,000 rpm, the supernatant was transferred to the previous tube. 50 to 70 µl of 5M K₂CO₃ was added to the collected supernatant to neutralize the liquid. After the pH of the liquid had been confirmed to be neutral using litmus paper, the liquid volume was adjusted to 600 µl with sterile water to obtain the measurement sample. Roche Diagnostics nitric acid and urea/ammonia F-kits were used to measure nitric acid and urea, respectively. The method of Ameziane et al (see previous) was modified for measuring 2-OG content. 20 µl of the extracted sample was added to 475 µl of reaction liquid (0.1 M Tris-HCl (pH 8.5), 1.0 mM CaCl₂, 0.2 mM NADPH, 0.2 M NH₄Cl), absorbance was measured at 340 nm, and 5 µl (10 units) of glutamate dehydrogenase (GDH) was added and reacted for 10 minutes at 37°C. Absorbance was measured again at 340 nm, and 2-OG content was calculated from the difference between absorbance after addition of the enzyme liquid and absorbance before addition of the enzyme.

As a result, the transformed *Arabidopsis thaliana* had a 2-OG content 2.6 times, urea content 2 times and a nitrate content 1.2 times that of the untransformed *Arabidopsis thaliana*. Likewise, the transformed potato had a 2-OG content 1.7 times, urea content 1.5 times and a nitrate content 1.3 times that of the untransformed potato (Figures 6A through 6F).

2-OG content and nitrate content were also measured using plant tissue of *Arabidopsis thaliana* which had been cultured for 3 weeks in medium adjusted to nitrogen concentrations of 10, 5, 3, 0.3 and 0.1 mM. As a result, the difference between the 2-OG content of the transformed *Arabidopsis thaliana* and the 2-OG content of the untransformed *Arabidopsis thaliana* was observed to increase as the nitrogen content of the medium decreased (Figure 7). These results suggest that the introduced GDH functions to break down glutamate and synthesize 2-OG and ammonia. The accumulation of nitrate in the transformed *Arabidopsis thaliana* and untransformed *Arabidopsis thaliana* remained roughly steady in a medium containing 3 mM or more of nitrogen, and it was confirmed that under such conditions the accumulation in the transformed *Arabidopsis thaliana* was at most 1.3 times that in untransformed *Arabidopsis thaliana*. Under conditions of 0.3 and 0.1 mM nitrogen, however, it was 2.5 to 4.5 times (Figure 8). These results suggest that nitrate accumulates much more in transformed *Arabidopsis thaliana* leaf tissue than in untransformed *Arabidopsis thaliana*, reflecting an increased efficiency of nitrogen uptake.

### Example 8. Nitrogen assimilation-related enzyme activities in the leaf tissue of GDH gene-introduced Arabidopsis thaliana and potato

The enzyme activity of NADH-dependent glutamate synthase (NADH-GOGAT), nitrate reductase (NR) and glutamine synthase (GS) was measured in the leaf tissues (about 0.1 g) of transformed potatoes and transformed *Arabidopsis thaliana*. For the potatoes, the materials were the leaf tissues of vigorously growing plants that had been cultivated for 1 month in a mixed soil of 500 g Power Soil and 500 g vermiculite. The leaf tissues of the plants that had been raised for 3 weeks in PNS medium (containing 5 mM KNO₃) were used for the *Arabidopsis thaliana*. The crude enzyme solution was extracted by the methods of Groat et al (Ref: *Plant Physiol,* 1981, 67: 1198-1203). After the leaf tissue had been crushed with liquid nitrogen, 500 µl of ice-cooled extraction buffer (100 mM Mes-NaOH (pH 7.5), 100 mM sucrose, 2% (v/v) mercaptoethanol, 15% ethylene glycol, 0.1% PMSF) was added and mixed gently. Following centrifugation for 20 minutes at 0°C, 12,000 rpm, the supernatant was purified with Ultrafree (Biomax-10K, Millipore Co.). This was washed three times with the same buffer and dissolved in 100 µl of buffer to obtain the enzyme solution.

NADH-GOGAT activity was also measured by the methods of Groat et al (see previous). 500 µl of reaction solution (100 mM K-phosphate (pH 8.2), 100 µM NADH, 2.5 mM 2-oxoglutarate, 10 mM L-glutamine, 1 mM aminooxyacetate) was prepared, and 10 µl of the enzyme liquid was added thereto and mixed. Absorbance was measured for 3 minutes at 340 nm, and the decrease accompanying consumption of NADH was measured.

NR activity was measured by the methods of Ferrario-Mery et al (Ref: *Planta,* 197, 202:510-521). 490 µl reaction solution (50 mM Mops-KOH (pH 7.8), 1 mM NaF, 10 mM KNO₃, 0.17 mM NADH and 10 mM MgCl₂ or 5 mM EDTA) was prepared, and 10 µl of enzyme solution was added to each and mixed. 16 minutes later 500 µl of 1% sulfonyl amide (in 3N HCl) was added, and then 10 µl of 2% N-naphthyl-1-ethylene diamine dichlorohydrate was added. Following centrifugation at 12,000 rpm for 5 minutes, absorbance was measured at 540 nm. The value from the reaction with EDTA added was subtracted from the value from the reaction with MgCl₂ added to obtain NR activity.

GS activity was also measured by the methods of Ferrario-Mery et al (see previous). 20 µl of enzyme solution was added to 480 µl of reaction liquid (80 mM glutamate, 20 mM MgSO₄, 1 mM EDTA, 100 mM tricine, 6 mM hydroxylamine, 8 mM ATP), and reacted for 15 minutes at 30°C. 500 µl of 0.37 M FeCl₃, 0.2 M of trichloroacetic acid and a 0.67 N HCl solution were added to stop the reaction, and absorbance was measured at 540 nm.

The protein concentration of the extracted enzyme solution was measured by the Bradford method using bovine serum albumin (BSA) as a standard protein. As a result, the transformed *Arabidopsis thaliana* had 1.38 times the NADH-GOGAT activity, 1.40 times the NR activity and 1.25 times the GS activity of the untransformed *Arabidopsis thaliana* (Table 3). The transformed potato also had 1.33 times the NADH-GOGAT activity, 1.36 times the NR activity and 1.01 times the GS activity of the untransformed potato (Table 4). These results show that not only do transformed *Arabidopsis thaliana* and the transformed potatoes have greater nitrogen uptake efficiency than non-transformants, but enzymes involved in nitrogen metabolism are also activated, and the nitrogen metabolism efficiency was also increased in the transformed *Arabidopsis thaliana* and the transformed potatoes.

**Table 3. Enzyme activities in the transformed Arabidopsis thaliana (Mtd 3)**

| | NADH-GOGAT | NR | GS |
|---|---|---|---|
| Non-transformant (A) | 482.4 ± 86.8 | 1.88 ± 0.22 | 188.3 ± 24.9 |
| Transformant (B) | 667.5 ± 107.5 | 2.63 ± 0.37 | 235.7 ± 28.4 |
| Ratio (B/A) | 1.38 | 1.40 | 1.25 |

| | | | |
|---|---|---|---|
| (units/mg protein) | | | |

**Table 4. Enzyme activities in the transformed potato (Mtd 8)**

| | NADH-GOGAT | NR | GS |
|---|---|---|---|
| Non-transformant (A) | 38.6 ± 4.1 | 0.21 ± 0.04 | 99.0 ± 20.7 |
| Transformant (B) | 51.5 ± 2.4 | 0.29 ± 0.03 | 99.6 ± 8.5 |
| Ratio (B/A) | 1.33 | 1.36 | 1.01 |

| | | | |
|---|---|---|---|
| (units/mg protein) | | | |

### Example 9. Yield survey of transformed potato

The yields of the transformed potatoes were studied. Seed potatoes from the transformed potatoes and the untransformed potatoes were used in the yield survey. Growth, tuber weight, number of tubers and the like were determined under cultivation conditions with differing nitrogen concentrations. Two groups were set up―one is for standard conditions of 3 kg of Power Soil (total nitrogen 1.2 kg) in an 8.5-liter pot and the other is for low-nitrogen conditions of 300 g of Power Soil (total nitrogen 0.12 kg) in a No.7 pot, with the remainder of the soil being made up of vermiculate (without nutrients). The potatoes were harvested 3 months after being seeded, and above-ground weights, tuber weights and tuber numbers were measured.

In the 1.2 g nitrogen group, the transformed potatoes had 1.12 to 1.13 times the tuber weight and 1.09 to 1.23 times the number of tubers of the untransformed potatoes (Table 5). In the 0.12 g nitrogen group (nitrogen-limited conditions), the transformed potatoes had 1.29 to 1.35 times the tuber weight and 1.24 to 1.56 the number of tubers of the untransformed potatoes (Table 6).

**Table 5. Yield survey of transformed potatoes in 1.2 g nitrogen group (n ≥ 8)**

| | Non-transformant | Transformant | |
|---|---|---|---|
| | | Mtd 3 | Mtd 8 |
| Above-ground weight (g) | 8.2 ± 1.9 (1.00) | 7.8 ± 2.0 (0.95) | 8.6 ± 0.8 (1.05) |
| Tuber weight (g) | 217.6 ± 19.3 (1.00) | 244.1 ± 20.4 (1.12) | 246.1 ± 21.0 (1.13) |
| Number of tubers | 6.5 ± 1.7 (1.00) | 7.1 ± 2.8 (1.09) | 8.0 ± 2.1 (1.23) |

**Table 6. Yield survey of transformed potatoes in 0.12 g nitrogen group (n ≥ 8)**

| | Non-transformant | Transformant | |
|---|---|---|---|
| | | Mtd 1 | Mtd 8 |
| Above-ground weight (g) | 11.9 ± 2.1 (1.00) | 14.0 ± 4.0 (1.18) | 13.5 ± 2.4 (1.13) |
| Tuber weight (g) | 41.0 ± 7.0 (1.00) | 52.7 ± 7.9 (1.29) | 55.4 ± 6.5 (1.35) |
| Number of tubers | 2.5 ± 0.5 (1.00) | 3.9 ± 1.0 (1.56) | 3.1 ± 1.1 (1.24) |

### Example 10. Growth survey of transformed Arabidopsis thaliana

Growth was studied using transformed *Arabidopsis thaliana* (Mtd 3). The *Arabidopsis thaliana* were sown on PNS medium (nitrogen source: nitrate nitrogen only) supplemented with varying KNO₃ concentrations of 0.1, 0.3, 3 and 5 mM and 1% sucrose using 8 cm Thermo Petri dishes, 8 plants per dish. 3 weeks after sowing the above-ground weights were measured. The data represent the total above-ground weights of all 8 plants, presented as the mean and deviation for 3 Petri dishes in each group. As a result, the transformed *Arabidopsis thaliana* grew better under low-nitrogen conditions than did the untransformed *Arabidopsis thaliana*, exhibited a growth 1.1 to 1.14 times that of the untransformed *Arabidopsis thaliana* as fresh weight (Table 7).

**Table 7. Fresh weights (g) of transformed Arabidopsis thaliana and untransformed Arabidopsis thaliana grown in PNS medium (with 1% sucrose) comprising various KNO₃ concentrations**

| | | | | |
|---|---|---|---|---|
| KNO₃ concentration | 0.1 mM | 0.5 mM | 1.0 mM | 5.0 mM |
| Non-transformant (A) | 0.0704± 0.0073 | 0.1265± 0.0054 | 0.2026± 0.0037 | 0.5895± 0.0032 |
| | | | | |
| Transformant (B) | 0.0800± 0.0073 | 0.1441 ± 0.0054 | 0.2232± 0.0037 | 0.6101 ± 0.0032 |
| Ratio (B/A) | 1.14 | 1.14 | 1.10 | 1.03 |

### Example 11. Foliar application of an aqueous proline solution to potato plants

The effects of foliar application of a proline solution on growth and yield were investigating using potatoes (May Queen cultivar). Potato tubers were placed in No.7 pots containing 1.5 kg of Power Soil (Kureha Chemical). The nitrogen fertilizer content of the Power Soil was shown in Table 8. Under normal cultivation about 5 g of nitrogen fertilizer is used as base fertilizer, while in this cultivation test cultivation was under low-nitrogen conditions.

Three foliar application groups were prepared―a spreader group (Approach B1, Kao), a urea group (spreader + 0.87 mM urea) and a proline group (spreader + 1.75 mM proline), and about 20 ml per plant was sprayed once a week beginning about a month after planting (immediately before flowering). The leaves were sprayed 6 times (6 weeks), and harvested 3 months after planting.

**Table 8. Nitrogen fertilization conditions for potato cultivation test**

| | |
|---|---|
| Total nitrogen | 0.6 g |
| •Nitrate-nitrogen | 0.075 g |
| •Ammonia-nitrogen | 0.525 g |

### Example 12. Measurement of free proline content and 2-oxoglutarate (2-OG) content of potato leaf tissues after foliar application of proline

After potato leaf surfaces had been sprayed with aqueous solutions of a spreader, urea and proline, the free proline content and 2-OG content were measured over time. The 3^{rd} leaf from the apices was sampled 1, 3, 5 and 8 hours after foliar application, and washed thoroughly with water as the samples. After crushing with liquid nitrogen, 80% ethanol, which had been heated to 80°C and was equal to 5 times the fresh weight was added, and incubated for 20 minutes at 80°C. Following centrifugation for 10 minutes at 12,000 rpm, the supernatant was transferred to a separate tube. 80% ethanol was added again, followed by incubation for 20 minutes at 80°C and centrifugation, and the supernatant was collected and extracted in the same way a total of 3 times. The ethanol extract was freeze dried, and 300 µl sterile water and 200 µl ethyl ether were added and mixed well and centrifuged for 10 minutes at 12,000 rpm. After removal of the upper, ethyl ether layer, 200 µl of the water layer was transferred to a separate tube and once more freeze dried. After being dissolved in 200 µl of 0.02 N hydrochloric acid, this was filtered with a 0.22 µm filter. The resulting extract was analyzed with a Hitachi highspeed amino acid analyzer (L8800). The 2-OG content was also measured using similar plant samples. The extraction and the determination of the content of 2-OG was conducted according to the methods similar to those described in Example 7.

As a result, no significant difference was observed among the three groups in proline content of the leaf tissue one hour after foliar application, but 5 hours after application the free proline content of the leaf tissue was significantly greater in the proline application group (Figure 11). It was thought that the proline sprayed on the leaf surfaces had been incorporated into the leaf tissue. Similarly, when the 2-OG content in leaf tissue was measured no significant difference was observed among the three groups in 2-OG content in leaf tissue 1 hour after foliar application, but 24 hours after application the 2-OG content of the leaf tissue was significantly greater in the proline application group (Figure 12). It was thought that the proline sprayed on the leaf surfaces had been incorporated into the leaf tissue and then metabolized into 2-OG and entered to the TCA cycle.

### Example 13. Yield survey of potatoes after foliar application of proline

Aqueous solutions of a spreader, urea and proline were applied 6 times at one-week intervals to the leaf surfaces of potato plants 1 month after planting. These were cultivated for 3 months after planting, with 6 pots in each group and one stalk per plant. The tubers were harvested from each pot, and the tuber weight and number of tubers per pot was measured.

As a result, the tuber weight per pot was 145.8 g per pot in the spreader group, 140.1 g in the urea group and 158.4 g in the proline group, so the proline group had 1.1 times the tuber weight of the spreader group. The number of tubers was 8.2 in the spreader group, 6.8 in the urea group and 8.2 in the proline group, so no difference was seen in tuber numbers (Table 9). These results indicate that tuber weight is increased by foliar application of proline.

**Table 9. Yield of proline-sprayed potatoes (n = 6)**

| | Spreader | Urea | Proline |
|---|---|---|---|
| Total tuber weight (per plant) | 145. 8 ± 16.3 | 140.1 ± 10.6 | 158.4 ± 18.0 |
| Ratio | 1.00 | 0.96 | 1.10 |
| Total number of tubers (per plant) | 8.2 ± 1.9 | 6.8 ± 1.6 | 8.2 ± 1.0 |
| Ratio | 1.00 | 0.83 | 1.00 |

### Example 14. Production of transformed Arabidopsis thaliana into which an E. coli-derived aspartate aminotransferase (ECASPC) gene is introduced

An ECASPC gene was isolated based on the sequence data (GenBank accession NO: X03629) for an aspartate aminotransferase gene (ECASPC) already reported in *E. coli* (nucleotide sequence: SEQ ID NO: 19; amino acid sequence: SEQ ID NO: 20). A mitochondrial transit peptide sequence was added to this gene as described in Example 1 (mtdECASPC). This gene was substituted for the GUS region of the plant-transforming Ti-plasmid vector pBI121, and transformed into *Agrobacterium tumefaciens* EHA105. The mtdECASPC gene was introduced into *Arabidopsis thaliana* by the methods described in Example 3 using this *Agrobacterium.* This was then sowed in medium containing kanamycin, resistant individuals were selected (T1 generation), and a line in which kanamycin resistance and sensitivity were segregated 3:1 was obtained in the next generation (T2 generation). In the following generation, four lines with all individuals exhibiting kanamycin resistance (T3 generation) were selected (mtdECASPC2-2, mtdECASPC6-2, mtdECASPC8-1 and mtdECASPC9-1). The introduced gene was confirmed in the resulting transformants by genome PCR as in Example 4. Transcription and expression of the introduced gene were also analyzed by RT-PCR. RNA was extracted from the *Arabidopsis thaliana* leaf tissue using a Qiagen Plant RNeasy Mini-Kit. RT-PCR was performed using a TaKaRa RNA-PCR Kit and ECASPC-specific primers. The reaction consisted of 30 cycles of 1 minute at 94°C, 1 minute at 55°C and 2 minutes at 72°C, followed by 1% agarose gel electrophoresis and ethidium bromide staining.

As a result, the transformed *Arabidopsis thaliana* that exhibited kanamycin resistance produced a band of the same size as that obtained using as the template mtdECASPC plasmid DNA as a positive control (Figure 13). These results suggest that the mtdECASPC gene had been introduced into the genome of the transformed *Arabidopsis thaliana* which exhibited kanamycin resistance. An mtdECASPC-specific band was also confirmed by RT-PCR in the transformed *Arabidopsis thaliana* (Figure 14). Since this band was not seen with untransformed *Arabidopsis thaliana* or when RNA without the reverse transcription was used as the template, it appears that the genome was not contaminated. That is, these results suggest that the introduced mtdECASPC gene was being transcribed within the transformed *Arabidopsis thaliana*.

### Example 15. Growth survey of ECASPC gene-transformed Arabidopsis thaliana under nitrogen-limited conditions

Growth was surveyed as in Example 10 using the resulting transformants. PNS having nitrate nitrogen alone as the nitrogen source was used. The KNO₃ concentrations of the media were 5 mM and 10 mM, each medium was supplemented with 1% sucrose. The seeds were sterilely sown, and 10 plants with uniform growth were allowed to remain after 1 week. After 2 more weeks (3 weeks after sowing), the above-ground weights of the 10 plants were measured. The means and deviations for 2 plates are shown in Table 10. As a result, it was shown that *Arabidopsis thaliana* containing the introduced mtdECASPC gene had much greater above-ground weights than the untransformed *Arabidopsis thaliana* into which the gene had not been introduced, by a factor of 1.18 to 1.28 in the 5 mM nitrogen group and 1.12 to 1.38 in the 10 mM nitrogen group.

**Table 10. Growth survey of Arabidopsis thaliana with introduced mtdECASPC gene**

| | 5 mM | 10 mM |
|---|---|---|
| Non-transformant | 335.6 ± 10.5 | 352.2 ± 25.5 |
| Transformant | | |
| mtdECASPC2-2 | 405.4 ± 0.8 | 394.7 ± 10.3 |
| mtdECASPC6-2 | 394.7 ± 7.7 | 436.4 ± 3.4 |
| mtdECASPC8-1 | 430.3 ± 5.8 | 484.6 ± 7.0 |
| mtdECASPC9-1 | 420.6 ± 13.2 | 467.5 ± 2.2 |

| | | |
|---|---|---|
| (mg, n=2) | | |

### Example 16. Measurement of 2-OG content of mtdECASPC gene-transfected Arabidopsis thaliana

2-OG content was measured using the four lines of mtdECASPC gene-transfected *Arabidopsis thaliana* selected in Example 14. The above-ground parts of plants grown for 2 weeks at 24°C with a 16-hour day length from seeds which were sown sterilely in PNS medium containing 5 mM KNO₃ and 1 % sucrose were used. 2-OG was extracted and its content measured by methods similar to those described in Example 7.

As a result, the 2-OG content of the above-ground tissue of the transformed *Arabidopsis thaliana* with the introduced mtdECASPC gene was greater by a factor of 1.27 to 1.93 than that of the untransformed *Arabidopsis thaliana* into which the gene had not been introduced. The results are extremely similar to the results obtained for transformed plants with the introduced GDH gene.

**Table 11. 2-OG content of mtdECASPC gene-transfected Arabidopsis thaliana**

| | 2-OG content (µmol/g.F.W.) | Ratio |
|---|---|---|
| Non-transformant | | |
| Transformant | | |
| mtdECASPC2-2 | 0.254 ± 0.029 | 1.35 |
| mtdECASPC6-2 | 0.363 ± 0.085 | 1.93 |
| mtdECASPC8-1 | 0.239 ± 0.066 | 1.27 |
| mtdECASPC9-1 | 0.268 ± 0.080 | 1.42 |

| | | |
|---|---|---|
| (n>3) | | |

## Claims

1. A method for producing a plant exhibiting improved growth and/or improved yield under cultivation conditions where nitrogen is limited to the level below the ordinary cultivation conditions, comprising increasing the 2-oxoglutarate (2-OG) content of the plant.

2. A method for producing a plant exhibiting improved growth and/or yield under cultivation conditions where nitrogen is limited to the level below the ordinary, cultivation conditions, comprising introducing a glutamate dehydrogenase (GDH) gene into a plant and expressing the gene in a plant body, thereby increasing 2-OG content of the plant.

3. A method for producing a plant exhibiting improved growth and/or yield under cultivation conditions where nitrogen is limited to the level below the ordinary cultivation conditions, comprising spraying proline on a leaf of a plant, thereby increasing the 2-OG content of the plant.

4. A method for producing a plant exhibiting improved growth and/or yield under cultivation conditions where nitrogen is limited below the level of the ordinary cultivation conditions, comprising introducing an ECASPC gene into a plant and expressing the gene in the plant, thereby increasing 2-OG content of the plant.

5. A method for cultivating a plant having increased 2-OG content, comprising cultivating a plant under nitrogen-limited cultivation conditions.

6. A method for cultivating a plant into which a glutamate dehydrogenase (GDH) gene is introduced and having increased 2-OG content, comprising cultivating the plant under nitrogen-limited cultivation conditions.

7. A method for cultivating a plant which is received foliar application of proline and having increased 2-OG content, comprising cultivating the plant under nitrogen-limited cultivation conditions.

8. A method for cultivating a plant into which a ECASPC gene is introduced and having increased 2-OG content, comprising cultivating the plant under nitrogen-limited cultivation conditions.

9. The method according to any one of Claims 1 to 8, wherein growth and/or yield equal to or greater than growth and/or yield obtained under cultivation conditions with standard application amount of nitrogen fertilizer is obtained under cultivation conditions in which the nitrogen amount is in the range between below the lower limit and 1/10-fold the lower limit of the standard application amount of nitrogen fertilizer.

10. The method according to Claims 6 to 8, wherein the nitrogen-limited cultivation conditions are conditions in which the application amount of nitrogen fertilizer is in the range between below the lower limit and 1/10 the lower limit of the standard application amount of nitrogen fertilizer.


SEQUENCE LISTING

<110>  Ajinomoto Co., Inc.

<120>  A method of improving growth and yield of plants under reduced
       nitrogen

<130>  OP05262

<150>  JP 2003-198559
<151>  2003-07-17

<160>  20

<170>  PatentIn version 3.2

<210>  1
<211>  2334
<212>  DNA
<213>  Aspergillus nidulans

<220>
<221>  CDS
<222>  (400)..(445)

<220>
<221>  CDS
<222>  (499)..(774)

<220>
<221>  CDS
<222>  (828)..(1882)

<400>  1
ttagccctcc ctttccgatc ctatggttcc tccgatcctg accctggttg accagtgatc      60

cggggccttc gagcctttcc accgtgccag ccgactttcc cgccagattt tagtcgcctc     120

ccgtggccgc acccagggat cttctcgtcc gactccgggc agtatactca ttcgccggcc     180

tccgccaggc cgccgtccgc ctctccccac cacccgtctc ctctgtcata cttaaacccg     240

gtcgtgtgcc tctcttggac ggtctctttt tttttcttat ccatcagagc cctttgcttc     300

tctgctgctt caagtgcttc aattgtcttc tcgcgggctc agcgcattct tattaccttt     360

gccacattac cttatcagca ataagcttct cgcgcaaaa atg tct aac ctt ccc        414
                                           Met Ser Asn Leu Pro
                                           1               5

gtt gag ccc gag ttc gag cag gcc tac aag g gtacgaccac cgttaatgaa       465

Val Glu Pro Glu Phe Glu Gln Ala Tyr Lys
                10                  15

tggccaatga tcacagctaa tgttggcgcg cag ag  ctt gcg tcg acc ctc gag      518
                                      Glu Leu Ala Ser Thr Leu Glu
                                                          20

aac tcc acc ctc ttt gag cag cac cct gaa tac cga cgg gct ctc cag      566
Asn Ser Thr Leu Phe Glu Gln His Pro Glu Tyr Arg Arg Ala Leu Gln
        25                  30                  35

gtc gtc tcc gtt ccc gag cgc gtt atc cag ttc cgt gtc gtt tgg gag      614
Val Val Ser Val Pro Glu Arg Val Ile Gln Phe Arg Val Val Trp Glu
    40                  45                  50

aac gac aag ggc gag gtt cag atc aac cgc ggt tac cgt gtt cag ttc      662
Asn Asp Lys Gly Glu Val Gln Ile Asn Arg Gly Tyr Arg Val Gln Phe
55                  60                  65                  70

aac tcc gct ctc ggt ccc tac aag ggt ggt ctc cgt ttc cac ccc tcc      710
Asn Ser Ala Leu Gly Pro Tyr Lys Gly Gly Leu Arg Phe His Pro Ser
                75                  80                  85

gtc aac ctt tct atc ctg aag ttc ctt ggc ttc gag cag atc ttc aaa      758
Val Asn Leu Ser Ile Leu Lys Phe Leu Gly Phe Glu Gln Ile Phe Lys
            90                  95                  100

aat gct ctc aca gga c gtgcgtaacc gttacttcat tggatgtttg ccaagagtac      814
Asn Ala Leu Thr Gly
        105

taattggtat tag ta  aac atg ggt ggt ggc aag ggt ggt tcc gac ttc      862
                Leu Asn Met Gly Gly Gly Lys Gly Gly Ser Asp Phe
                        110                 115

gac ccc aag ggc aag tct gac tct gaa att cgt cgc ttc tgt acc gct      910
Asp Pro Lys Gly Lys Ser Asp Ser Glu Ile Arg Arg Phe Cys Thr Ala
120                 125                 130                 135

ttc atg act gag ctc tgc aag cac atc ggc gcg gac act gac ctt ccc      958
Phe Met Thr Glu Leu Cys Lys His Ile Gly Ala Asp Thr Asp Leu Pro
                140                 145                 150

gct ggt gat atc ggt gtt act ggc cgt gag gtt ggt ttc ctt ttc ggc      1006
Ala Gly Asp Ile Gly Val Thr Gly Arg Glu Val Gly Phe Leu Phe Gly
            155                 160                 165

cag tac cgc agg atc cgc aac cag tgg gag ggt gtt ctc act ggc aag      1054
Gln Tyr Arg Arg Ile Arg Asn Gln Trp Glu Gly Val Leu Thr Gly Lys
        170                 175                 180

ggt ggc agc tgg ggt ggt agc ttg atc cgc cct gaa gcc act gga tac      1102

Gly Gly Ser Trp Gly Gly Ser Leu Ile Arg Pro Glu Ala Thr Gly Tyr
    185                 190                 195

ggt gtt gtc tac tac gtt cag cac atg atc aag cac gtt acc ggt gga 1150
Gly Val Val Tyr Tyr Val Gln His Met Ile Lys His Val Thr Gly Gly
200                 205                 210                 215

aag gag tcc ttc gca ggc aag cgt gtc gcc atc tcc ggc tcc ggt aac 1198
Lys Glu Ser Phe Ala Gly Lys Arg Val Ala Ile Ser Gly Ser Gly Asn
                220                 225                 230

gtt gcc cag tac gcc gct ctc aag gtc atc gag ctc ggt ggt tcc gtt 1246
Val Ala Gln Tyr Ala Ala Leu Lys Val Ile Glu Leu Gly Gly Ser Val
            235                 240                 245

gtc tcc ctt tcc gac tcc aag ggc tct ctc att gtc aag gat gag tcc 1294
Val Ser Leu Ser Asp Ser Lys Gly Ser Leu Ile Val Lys Asp Glu Ser
        250                 255                 260

gct tct ttc acc cct gaa gag atc gcc ctc att gcc gac ctc aag gtt 1342
Ala Ser Phe Thr Pro Glu Glu Ile Ala Leu Ile Ala Asp Leu Lys Val
    265                 270                 275

gcc cgc aag caa ctc tcc gag ctc gcc acc tcc tcc gct ttc gcc ggc 1390
Ala Arg Lys Gln Leu Ser Glu Leu Ala Thr Ser Ser Ala Phe Ala Gly
280                 285                 290                 295

aag ttc acc tac atc ccc gat gct cgc cct tgg acc aac att ccc ggc 1438
Lys Phe Thr Tyr Ile Pro Asp Ala Arg Pro Trp Thr Asn Ile Pro Gly
                300                 305                 310

aag ttc gag gtt gct ctc cct tct gcc act cag aac gaa gtc tcc ggc 1486
Lys Phe Glu Val Ala Leu Pro Ser Ala Thr Gln Asn Glu Val Ser Gly
            315                 320                 325

gag gaa gcc gag cac ctc atc aag tcc ggt gtc cgc tat att gct gag 1534
Glu Glu Ala Glu His Leu Ile Lys Ser Gly Val Arg Tyr Ile Ala Glu
        330                 335                 340

ggt tcc aac atg ggt tgc acc cag gcc gcc atc gac atc ttt gag gct 1582
Gly Ser Asn Met Gly Cys Thr Gln Ala Ala Ile Asp Ile Phe Glu Ala
    345                 350                 355

cac cgc aac gcc aac ccc ggc gat gcc atc tgg tac gcc cct ggt aaa 1630
His Arg Asn Ala Asn Pro Gly Asp Ala Ile Trp Tyr Ala Pro Gly Lys
360                 365                 370                 375

gcc gcc aac gct ggt ggt gtc gcc gtc tct ggt ctt gag atg gct cag 1678
Ala Ala Asn Ala Gly Gly Val Ala Val Ser Gly Leu Glu Met Ala Gln
                380                 385                 390

aac tct gct cgt ctc tcc tgg aca tcc gag gag gtc gat gct cgc ctc 1726

Asn Ser Ala Arg Leu Ser Trp Thr Ser Glu Glu Val Asp Ala Arg Leu
            395                 400                 405

aag ggc atc atg gag gac tgc ttc aag aac ggt ctc gag act gct cag 1774
Lys Gly Ile Met Glu Asp Cys Phe Lys Asn Gly Leu Glu Thr Ala Gln
        410                 415                 420

aag ttc gct act cct gcc aag ggc gtc ctg cct tcc ctc gtc acc ggt 1822
Lys Phe Ala Thr Pro Ala Lys Gly Val Leu Pro Ser Leu Val Thr Gly
    425                 430                 435

tcc aac att gcc ggt ttc acc aag gtc gcc gag gcc atg aag gac cag 1870
Ser Asn Ile Ala Gly Phe Thr Lys Val Ala Glu Ala Met Lys Asp Gln
440                 445                 450                 455

ggt gac tgg tgg tgaattagcc ccgtctccta attttgatgt tgcatgatga 1922
Gly Asp Trp Trp

tgattcctac gtttgttcca gcctttcggg ttttggtctt gttatgatct taccattctt 1982

agcgtggata gaggcgctgg attcgtttct actatgcttt tacctgtaga tagacctttt 2042

ttgcatagtt atagtatatt ataccataga cgactttatt cttacccttt tccttagtaa 2102

tgtgcaagca ctatccacat tctgaatagc ccagtatgag atttgtactc aaaaaaactt 2162

aatgcccaga aaacactcgc aataagcacg gccgtgtcgc gttgtgttcc ttgagcctca 2222

acagttcgaa ttaattatcg tggttggctc aggcctttat catgtgacta gactaaggat 2282

tcacgtgatt agctcttgct tccacgcttc ggaatctaca tgaatgaagc tt 2334

<210> 2
<211> 459
<212> PRT
<213> Aspergillus nidulans

<400> 2

Met Ser Asn Leu Pro Val Glu Pro Glu Phe Glu Gln Ala Tyr Lys Glu
1               5                   10                  15

Leu Ala Ser Thr Leu Glu Asn Ser Thr Leu Phe Glu Gln His Pro Glu
            20                  25                  30

Tyr Arg Arg Ala Leu Gln Val Val Ser Val Pro Glu Arg Val Ile Gln
        35                  40                  45

Phe Arg Val Val Trp Glu Asn Asp Lys Gly Glu Val Gln Ile Asn Arg
    50                  55                  60

Gly Tyr Arg Val Gln Phe Asn Ser Ala Leu Gly Pro Tyr Lys Gly Gly
65                  70                  75                  80

Leu Arg Phe His Pro Ser Val Asn Leu Ser Ile Leu Lys Phe Leu Gly
                85                  90                  95

Phe Glu Gln Ile Phe Lys Asn Ala Leu Thr Gly Leu Asn Met Gly Gly
            100                 105                 110

Gly Lys Gly Gly Ser Asp Phe Asp Pro Lys Gly Lys Ser Asp Ser Glu
        115                 120                 125

Ile Arg Arg Phe Cys Thr Ala Phe Met Thr Glu Leu Cys Lys His Ile
    130                 135                 140

Gly Ala Asp Thr Asp Leu Pro Ala Gly Asp Ile Gly Val Thr Gly Arg
145                 150                 155                 160

Glu Val Gly Phe Leu Phe Gly Gln Tyr Arg Arg Ile Arg Asn Gln Trp
                165                 170                 175

Glu Gly Val Leu Thr Gly Lys Gly Gly Ser Trp Gly Gly Ser Leu Ile
            180                 185                 190

Arg Pro Glu Ala Thr Gly Tyr Gly Val Val Tyr Tyr Val Gln His Met
        195                 200                 205

Ile Lys His Val Thr Gly Gly Lys Glu Ser Phe Ala Gly Lys Arg Val
    210                 215                 220

Ala Ile Ser Gly Ser Gly Asn Val Ala Gln Tyr Ala Ala Leu Lys Val
225                 230                 235                 240

Ile Glu Leu Gly Gly Ser Val Val Ser Leu Ser Asp Ser Lys Gly Ser
                245                 250                 255

Leu Ile Val Lys Asp Glu Ser Ala Ser Phe Thr Pro Glu Glu Ile Ala
            260                 265                 270

Leu Ile Ala Asp Leu Lys Val Ala Arg Lys Gln Leu Ser Glu Leu Ala
        275                 280                 285

Thr Ser Ser Ala Phe Ala Gly Lys Phe Thr Tyr Ile Pro Asp Ala Arg
    290                 295                 300

Pro Trp Thr Asn Ile Pro Gly Lys Phe Glu Val Ala Leu Pro Ser Ala
305                 310                 315                 320

Thr Gln Asn Glu Val Ser Gly Glu Glu Ala Glu His Leu Ile Lys Ser
                325                 330                 335

Gly Val Arg Tyr Ile Ala Glu Gly Ser Asn Met Gly Cys Thr Gln Ala
            340                 345                 350

Ala Ile Asp Ile Phe Glu Ala His Arg Asn Ala Asn Pro Gly Asp Ala
        355                 360                 365

Ile Trp Tyr Ala Pro Gly Lys Ala Ala Asn Ala Gly Gly Val Ala Val
    370                 375                 380

Ser Gly Leu Glu Met Ala Gln Asn Ser Ala Arg Leu Ser Trp Thr Ser
385                 390                 395                 400

Glu Glu Val Asp Ala Arg Leu Lys Gly Ile Met Glu Asp Cys Phe Lys
                405                 410                 415

Asn Gly Leu Glu Thr Ala Gln Lys Phe Ala Thr Pro Ala Lys Gly Val
            420                 425                 430

Leu Pro Ser Leu Val Thr Gly Ser Asn Ile Ala Gly Phe Thr Lys Val
        435                 440                 445

Ala Glu Ala Met Lys Asp Gln Gly Asp Trp Trp
    450                 455

<210> 3
<211> 2571
<212> DNA
<213> Aspergillus awamori

<220>
<221> CDS
<222> (741)..(785)

<220>
<221> CDS
<222> (850)..(1142)

<220>
<221> CDS
<222> (1472)..(2243)

<400> 3
tctagattgc gacggcgtat tgcttatcct tagtaggact ccctaatgga ttccgagcaa 60

gaaaagactg tttggcgtgt accaatggct catagtacca gcaagagaag aattttctct 120

ctcgcttcga gaaagcaatc aaaaaaaaat cctatcctac cctaccctac cctaatactt 180

ccattgccac ccgattcctc ccgatagtag agcgggcgac tgccatttgg cgggcgggcc 240

agcggattcc cgccgataga taacgggcag attctgtgac ctcaaactat cgactaacag 300

cccgaacttc ggcggccacc gccaaacccg ccccggaagc cggcctcatt tgccgtttgg 360

gcgtgccagg aaatgccgcc tgcagcggag actccctagt gtggtctgtg ttgcctgtgt 420

cgtctgtgta gtatactagt tactagtcta ctactgtaca gtggatggcc tgaggggggg 480

actttatgtc cgactccggc tgttctcctc cctctatcca ctctaccctc ttccctctct 540

tctgtctttc tccccgctct cgcccctccc ctcctcgaaa acataaatcg gcctttcccc 600

ctcgccatct tcttcttctt ctccctctcc tttctctttc ttcttcagac tacttctctt 660

tctttcatct tttctctata ttcctgtttt cctagatacc ccagttaaaa aagttctctc 720

aatcaatcct ccccttcaga atg tct aac ctt cct cac gag ccc gag ttc gag 773
Met Ser Asn Leu Pro His Glu Pro Glu Phe Glu
1               5                   10

cag gcc tac aag ggtatgttcc attgcccctc cgaaattgat gatggaaaaa 825
Gln Ala Tyr Lys
            15

aaattctaac aacatcctct taca gag ctt gcc tcg acc ctt gag aac tcc 876
Glu Leu Ala Ser Thr Leu Glu Asn Ser
                20

acc ctc ttc cag aag aac ccc gaa tac cgc aag gcc ctt gct gtc gtc 924
Thr Leu Phe Gln Lys Asn Pro Glu Tyr Arg Lys Ala Leu Ala Val Val
25                  30                  35                  40

tcc gtc ccc gag cgt gtc atc cag ttc cgt gtc gtc tgg gag gat gat 972
Ser Val Pro Glu Arg Val Ile Gln Phe Arg Val Val Trp Glu Asp Asp
                45                  50                  55

gcc ggc aac gtc cag gtc aac cgc ggt ttc cgt gtc cag ttc aac agc 1020
Ala Gly Asn Val Gln Val Asn Arg Gly Phe Arg Val Gln Phe Asn Ser
            60                  65                  70

gcc ctc ggt ccc tac aag ggt ggt ctt cgt ttc cac ccc tcc gtc aac 1068
Ala Leu Gly Pro Tyr Lys Gly Gly Leu Arg Phe His Pro Ser Val Asn
        75                  80                  85

ttg tcc atc ctc caa gtt cct tgg ttt cga gca gat ctt caa gaa tgc 1116
Leu Ser Ile Leu Gln Val Pro Trp Phe Arg Ala Asp Leu Gln Glu Cys
    90                  95                  100

tct cac tgg cct gaa cat ggg tgg tg gtaagggtgg ttccgacttc 1162
Ser His Trp Pro Glu His Gly Trp Trp
105                 110

gaccccaagg gcaagtccga caacgagatc cgtcgcttct gtgtttcctt catgaccgag 1222

ctctgcaagc acatcggtgc cgacactgat gttcccgctg gtgacatcgg tgtcaccggt 1282

cgtgaggtcg gtttcctctt cggccagtac cgcaagatcc gcaaccagtg ggagggtgtt 1342

ctcaccggta agggtggcag ctggggtggt tccctcatcc gccctgaggc caccggttac 1402

ggtgttgtct acgtatgtca attcctcttc ttatgattat ctatgtataa cagcgactaa 1462

cgcgtaaca g tac gtc gag cac atg att gct cac gcc acc aac ggc cag 1511
Tyr Val Glu His Met Ile Ala His Ala Thr Asn Gly Gln
    115                 120                 125

gag tcc ttc aag ggc aag cgc gtt gcc atc tcc ggt tcc ggt aac gtt 1559
Glu Ser Phe Lys Gly Lys Arg Val Ala Ile Ser Gly Ser Gly Asn Val
            130                 135                 140

gcc cag tac gcc gcc ctc aag gtc att gag ctc ggc ggt tcc gtc gtc 1607
Ala Gln Tyr Ala Ala Leu Lys Val Ile Glu Leu Gly Gly Ser Val Val
        145                 150                 155

tcc ctg agc gac acg cag ggc tcc ctc atc atc aac ggc gag ggt agc 1655
Ser Leu Ser Asp Thr Gln Gly Ser Leu Ile Ile Asn Gly Glu Gly Ser

160                 165                 170

ttc acc ccc gag gag atc gag ctc atc gct cag acc aag gtc gag cgc      1703
Phe Thr Pro Glu Glu Ile Glu Leu Ile Ala Gln Thr Lys Val Glu Arg
175                 180                 185                 190

aac gag ctc gcc agc atc gtc ggt gct gct ccc ttc agc gac gcc aac      1751
Asn Glu Leu Ala Ser Ile Val Gly Ala Ala Pro Phe Ser Asp Ala Asn
                195                 200                 205

aag ttc aag tac att gct ggt gcc cgc ccc tgg gtt cac gtc ggc aag      1799
Lys Phe Lys Tyr Ile Ala Gly Ala Arg Pro Trp Val His Val Gly Lys
            210                 215                 220

gtc gac gtc gct ctc ccc tcc gct acc cag aac gaa gtt tcc ggc gag      1847
Val Asp Val Ala Leu Pro Ser Ala Thr Gln Asn Glu Val Ser Gly Glu
        225                 230                 235

gag gcc cag gtc ctc atc aac gct ggc tgc aag ttc atc gcc gag ggt      1895
Glu Ala Gln Val Leu Ile Asn Ala Gly Cys Lys Phe Ile Ala Glu Gly
    240                 245                 250

tcc aac atg ggt tgc acc cag gag gcc atc gac acc ttc gag gcc cac      1943
Ser Asn Met Gly Cys Thr Gln Glu Ala Ile Asp Thr Phe Glu Ala His
255                 260                 265                 270

cgt acc gcc aac gct ggc gcg gct gcc atc tgg tac gcc ccc ggt aag      1991
Arg Thr Ala Asn Ala Gly Ala Ala Ala Ile Trp Tyr Ala Pro Gly Lys
                275                 280                 285

gcc gcc aac gcc ggt ggt gtc gct gtc tcc ggt ctg gag atg gct cag      2039
Ala Ala Asn Ala Gly Gly Val Ala Val Ser Gly Leu Glu Met Ala Gln
            290                 295                 300

aac tct gcc cgc ctc agc tgg act tct gag gag gtt gat gcc cgt ctt      2087
Asn Ser Ala Arg Leu Ser Trp Thr Ser Glu Glu Val Asp Ala Arg Leu
        305                 310                 315

aag gac atc atg cgc gac tgc ttc aag aac ggt ctt gag act gct cag      2135
Lys Asp Ile Met Arg Asp Cys Phe Lys Asn Gly Leu Glu Thr Ala Gln
    320                 325                 330

gag tac gcc acc ccc gct gag ggt gtc ctg cct tcc ctg gtg acc gga      2183
Glu Tyr Ala Thr Pro Ala Glu Gly Val Leu Pro Ser Leu Val Thr Gly
335                 340                 345                 350

tcc aac att gcc ggt ttc acc aag gtg gct gcc gcc atg aag gac cag      2231
Ser Asn Ile Ala Gly Phe Thr Lys Val Ala Ala Ala Met Lys Asp Gln
                355                 360                 365

ggt gac tgg tgg taaatgcgga aagccgcaaa cccccgcggc ttatgtcatg      2283
Gly Asp Trp Trp

            370

acgattatgt agtttgatgt tccctttcag cgcggatgga tagaggcgcc ggtgttttct 2343

tgctagttta gatggatgca taatgatatc cttttcttaa tcctcaaatt cttgtaattt 2403

gttgtatcaa tagtagataa tacaactgta gtcaactacc cttgcatctt cactatttgc 2463

agatgcattc atctctattc cgagcacatg cacaaaccca tgggaccgca gttcactagt 2523

acttagcctg ttatcttccc tctatcgcat cttaaacaac tatctaga 2571

<210> 4
<211> 370
<212> PRT
<213> Aspergillus awamori

<400> 4

Met Ser Asn Leu Pro His Glu Pro Glu Phe Glu Gln Ala Tyr Lys Glu
1               5                   10                  15

Leu Ala Ser Thr Leu Glu Asn Ser Thr Leu Phe Gln Lys Asn Pro Glu
            20                  25                  30

Tyr Arg Lys Ala Leu Ala Val Val Ser Val Pro Glu Arg Val Ile Gln
        35                  40                  45

Phe Arg Val Val Trp Glu Asp Asp Ala Gly Asn Val Gln Val Asn Arg
    50                  55                  60

Gly Phe Arg Val Gln Phe Asn Ser Ala Leu Gly Pro Tyr Lys Gly Gly
65                  70                  75                  80

Leu Arg Phe His Pro Ser Val Asn Leu Ser Ile Leu Gln Val Pro Trp
                85                  90                  95

Phe Arg Ala Asp Leu Gln Glu Cys Ser His Trp Pro Glu His Gly Trp
            100                 105                 110

Trp Tyr Val Glu His Met Ile Ala His Ala Thr Asn Gly Gln Glu Ser
        115                 120                 125

Phe Lys Gly Lys Arg Val Ala Ile Ser Gly Ser Gly Asn Val Ala Gln
    130                 135                 140

Tyr Ala Ala Leu Lys Val Ile Glu Leu Gly Gly Ser Val Val Ser Leu
145                 150                 155                 160

Ser Asp Thr Gln Gly Ser Leu Ile Ile Asn Gly Glu Gly Ser Phe Thr
                165                 170                 175

Pro Glu Glu Ile Glu Leu Ile Ala Gln Thr Lys Val Glu Arg Asn Glu
            180                 185                 190

Leu Ala Ser Ile Val Gly Ala Ala Pro Phe Ser Asp Ala Asn Lys Phe
        195                 200                 205

Lys Tyr Ile Ala Gly Ala Arg Pro Trp Val His Val Gly Lys Val Asp
    210                 215                 220

Val Ala Leu Pro Ser Ala Thr Gln Asn Glu Val Ser Gly Glu Glu Ala
225                 230                 235                 240

Gln Val Leu Ile Asn Ala Gly Cys Lys Phe Ile Ala Glu Gly Ser Asn
                245                 250                 255

Met Gly Cys Thr Gln Glu Ala Ile Asp Thr Phe Glu Ala His Arg Thr
            260                 265                 270

Ala Asn Ala Gly Ala Ala Ala Ile Trp Tyr Ala Pro Gly Lys Ala Ala
        275                 280                 285

Asn Ala Gly Gly Val Ala Val Ser Gly Leu Glu Met Ala Gln Asn Ser
    290                 295                 300

Ala Arg Leu Ser Trp Thr Ser Glu Glu Val Asp Ala Arg Leu Lys Asp
305                 310                 315                 320

Ile Met Arg Asp Cys Phe Lys Asn Gly Leu Glu Thr Ala Gln Glu Tyr
                325                 330                 335

Ala Thr Pro Ala Glu Gly Val Leu Pro Ser Leu Val Thr Gly Ser Asn
            340                 345                 350

Ile Ala Gly Phe Thr Lys Val Ala Ala Ala Met Lys Asp Gln Gly Asp
        355                 360                 365

Trp Trp
    370

<210>  5
<211>  27
<212>  DNA
<213>  Artificial

<220>
<223>  PCR primer

<400>  5
   tctagaatgt ctaaccccct tgttgag                                          27

<210>  6
<211>  27
<212>  DNA
<213>  Artificial

<220>
<223>  PCR primer

<400>  6
gagctctcac caccagtcac cctggtc                                          27

<210>  7
<211>  26
<212>  DNA
<213>  Artificial

<220>
<223>  PCR primer

<400>  7
ctgcagatga atgctttagc agcaac                                           26

<210>  8
<211>  26
<212>  DNA
<213>  Artificial

<220>
<223>  PCR primer

<400>  8
tctagataaa ccaagaagcc tagctg                                          26


<210>  9
<211>  26
<212>  DNA
<213>  Artificial

<220>
<223>  PCR primer

<400>  9
tctagaatga atgctttagc agcaac                                          26


<210>  10
<211>  31
<212>  DNA
<213>  Artificial

<220>
<223>  PCR primer

<400>  10
gggaaggttt agacattaaa ccaagaagcc t                                    31


<210>  11
<211>  30
<212>  DNA
<213>  Artificial

<220>
<223>  PCR primer

<400>  11
aggcttcttg gtttaatgtc taaccttccc                                      30


<210>  12
<211>  26
<212>  DNA
<213>  Artificial

<220>
<223>  PCR primer

<400>  12
gagctcttac gcctcccatc ctcgaa                                          26

<210>  13
<211>  27
<212>  DNA
<213>  Artificial

<220>
<223>  PCR primer

<400>  13
   tctagaatgt ctaaccccct tgttgag                                        27

<210>  14
<211>  27
<212>  DNA
<213>  Artificial

<220>
<223>  PCR primer

<400>  14
gagctctcac caccagtcac cctggtc                                         27

<210>  15
<211>  21
<212>  DNA
<213>  Artificial

<220>
<223>  PCR primer

<400>  15
cccctcggta tccaattaga g                                               21

<210>  16
<211>  24
<212>  DNA
<213>  Artificial

<220>
<223>  PCR primer

<400>  16
cggggggtgg gcgaagaact ccag                                            24

<210> 17
<211> 1380
<212> DNA
<213> Aspergillus nidulance

<400> 17
atgtctaacc ttcccgttga gcccgagttc gagcaggcct acaaggagct tgcgtcgacc 60

ctcgagaact ccaccctctt tgagcagcac cctgaatacc gacgggctct ccaggtcgtc 120

tccgttcccg agcgcgttat ccagttccgt gtcgtttggg agaacgacaa gggcgaggtt 180

cagatcaacc gcggttaccg tgttcagttc aactccgctc tcggtcccta caagggtggt 240

ctccgtttcc acccctccgt caacctttct atcctgaagt tccttggctt cgagcagatc 300

ttcaaaaatg ctctcacagg actaaacatg ggtggtggca agggtggttc cgacttcgac 360

cccaagggca agtctgactc tgaaattcgt cgcttctgta ccgctttcat gactgagctc 420

tgcaagcaca tcggcgcgga cactgacctt cccgctggtg atatcggtgt tactggccgt 480

gaggttggtt tccttttcgg ccagtaccgc aggatccgca accagtggga gggtgttctc 540

actggcaagg gtggcagctg gggtggtagc ttgatccgcc ctgaagccac tggatacggt 600

gttgtctact acgttcagca catgatcaag cacgttaccg gtggaaagga gtccttcgca 660

ggcaagcgtg tcgccatctc cggctccggt aacgttgccc agtacgccgc tctcaaggtc 720

atcgagctcg gtggttccgt tgtctccctt tccgactcca agggctctct cattgtcaag 780

gatgagtccg cttctttcac ccctgaagag atcgccctca ttgccgacct caaggttgcc 840

cgcaagcaac tctccgagct cgccacctcc tccgctttcg ccggcaagtt cacctacatc 900

cccgatgctc gcccttggac caacattccc ggcaagttcg aggttgctct cccttctgcc 960

actcagaacg aagtctccgg cgaggaagcc gagcacctca tcaagtccgg tgtccgctat 1020

attgctgagg gttccaacat gggttgcacc caggccgcca tcgacatctt tgaggctcac 1080

cgcaacgcca accccggcga tgccatctgg tacgcccctg gtaaagccgc caacgctggt 1140

ggtgtcgccg tctctggtct tgagatggct cagaactctg ctcgtctctc ctggacatcc 1200

gaggaggtcg atgctcgcct caagggcatc atggaggact gcttcaagaa cggtctcgag 1260

actgctcaga agttcgctac tcctgccaag ggcgtcctgc cttccctcgt caccggttcc 1320

aacattgccg gtttcaccaa ggtcgccgag gccatgaagg accagggtga ctggtggtga 1380

<210> 18
<211> 1383
<212> DNA
<213> Aspergillus awamori

<400> 18
atgtctaacc ttcctcacga gcccgagttc gagcaggcct acaaggagct tgcctcgacc 60

cttgagaact ccaccctctt ccagaagaac cccgaatacc gcaaggccct tgctgtcgtc 120

tccgtccccg agcgtgtcat ccagttccgt gtcgtctggg aggatgatgc cggcaacgtc 180

caggtcaacc gcggtttccg tgtccagttc aacagcgccc tcggtcccta caagggtggt 240

cttcgtttcc acccctccgt caacttgtcc atcctcaagt tccttggttt cgagcagatc 300

ttcaagaatg ctctcactgg cctgaacatg ggtggtggta agggtggttc cgacttcgac 360

cccaagggca agtccgacaa cgagatccgt cgcttctgtg tttccttcat gaccgagctc 420

tgcaagcaca tcggtgccga cactgatgtt cccgctggtg acatcggtgt caccggtcgt 480

gaggtcggtt tcctcttcgg ccagtaccgc aagatccgca accagtggga gggtgttctc 540

accggtaagg gtggcagctg gggtggttcc ctcatccgcc ctgaggccac cggttacggt 600

gttgtctact acgtcgagca catgattgct cacgccacca acggccagga gtccttcaag 660

ggcaagcgcg ttgccatctc cggttccggt aacgttgccc agtacgccgc cctcaaggtc 720

attgagctcg gcggttccgt cgtctccctg agcgacacgc agggctccct catcatcaac 780

ggcgagggta gcttcacccc cgaggagatc gagctcatcg ctcagaccaa ggtcgagcgc 840

aacgagctcg ccagcatcgt cggtgctgct cccttcagcg acgccaacaa gttcaagtac 900

attgctggtg cccgcccctg ggttcacgtc ggcaaggtcg acgtcgctct cccctccgct 960

acccagaacg aagtttccgg cgaggaggcc caggtcctca tcaacgctgg ctgcaagttc 1020

atcgccgagg gttccaacat gggttgcacc caggaggcca tcgacacctt cgaggcccac 1080

cgtaccgcca acgctggcgc ggctgccatc tggtacgccc ccggtaaggc cgccaacgcc 1140

ggtggtgtcg ctgtctccgg tctggagatg gctcagaact ctgcccgcct cagctggact 1200

tctgaggagg ttgatgcccg tcttaaggac atcatgcgcg actgcttcaa gaacggtctt 1260

gagactgctc aggagtacgc cacccccgct gagggtgtcc tgccttccct ggtgaccgga 1320

tccaacattg ccggtttcac caaggtggct gccgccatga aggaccaggg tgactggtgg 1380

taa                                                              1383

<210>  19
<211>  1191
<212>  DNA
<213>  Escherichia coli

<220>
<221>  CDS
<222>  (1)..(1188)

<400>  19
atg ttt gag aac att acc gcc gct cct gcc gac ccg att ctg ggc ctg      48
Met Phe Glu Asn Ile Thr Ala Ala Pro Ala Asp Pro Ile Leu Gly Leu
1               5                   10                  15

gcc gat ctg ttt cgt gcc gat gaa cgt ccc ggc aaa att aac ctc ggg      96
Ala Asp Leu Phe Arg Ala Asp Glu Arg Pro Gly Lys Ile Asn Leu Gly
            20                  25                  30

att ggt gtc tat aaa gat gag acg ggc aaa acc ccg gta ctg acc agc      144
Ile Gly Val Tyr Lys Asp Glu Thr Gly Lys Thr Pro Val Leu Thr Ser
        35                  40                  45

gtg aaa aag gct gaa cag tat ctg ctc gaa aat gaa acc acc aaa aat      192
Val Lys Lys Ala Glu Gln Tyr Leu Leu Glu Asn Glu Thr Thr Lys Asn
    50                  55                  60

tac ctc ggc att gac ggc atc cct gaa ttt ggt cgc tgc act cag gaa      240
Tyr Leu Gly Ile Asp Gly Ile Pro Glu Phe Gly Arg Cys Thr Gln Glu
65                  70                  75                  80

ctg ctg ttt ggt aaa ggt agc gcc ctg atc aat gac aaa cgt gct cgc      288
Leu Leu Phe Gly Lys Gly Ser Ala Leu Ile Asn Asp Lys Arg Ala Arg
                85                  90                  95

acg gca cag act ccg ggg ggc act ggc gca cta cgc gtg gct gcc gat      336
Thr Ala Gln Thr Pro Gly Gly Thr Gly Ala Leu Arg Val Ala Ala Asp
            100                 105                 110

ttc ctg gca aaa aat acc agc gtt aag cgt gtg tgg gtg agc aac cca      384
Phe Leu Ala Lys Asn Thr Ser Val Lys Arg Val Trp Val Ser Asn Pro
        115                 120                 125

agc tgg ccg aac cat aag agc gtc ttt aac tct gca ggt ctg gaa gtt      432
Ser Trp Pro Asn His Lys Ser Val Phe Asn Ser Ala Gly Leu Glu Val
    130                 135                 140

cgt gaa tac gct tat tat gat gcg gaa aat cac act ctt gac ttc gat      480

Arg Glu Tyr Ala Tyr Tyr Asp Ala Glu Asn His Thr Leu Asp Phe Asp
145                 150                 155                 160

gca ctg att aac agc ctg aat gaa gct cag gct ggc gac gta gtg ctg      528
Ala Leu Ile Asn Ser Leu Asn Glu Ala Gln Ala Gly Asp Val Val Leu
                165                 170                 175

ttc cat ggc tgc tgc cat aac cca acc ggt atc gac cct acg ctg gaa      576
Phe His Gly Cys Cys His Asn Pro Thr Gly Ile Asp Pro Thr Leu Glu
            180                 185                 190

caa tgg caa aca ctg gca caa ctc tcc gtt gag aaa ggc tgg tta ccg      624
Gln Trp Gln Thr Leu Ala Gln Leu Ser Val Glu Lys Gly Trp Leu Pro
        195                 200                 205

ctg ttt gac ttc gct tac cag ggt ttt gcc cgt ggt ctg gaa gaa gat      672
Leu Phe Asp Phe Ala Tyr Gln Gly Phe Ala Arg Gly Leu Glu Glu Asp
    210                 215                 220

gct gaa gga ctg cgc gct ttc gcg gct atg cat aaa gag ctg att gtt      720
Ala Glu Gly Leu Arg Ala Phe Ala Ala Met His Lys Glu Leu Ile Val
225                 230                 235                 240

gcc agt tcc tac tct aaa aac ttt ggc ctg tac aac gag cgt gtt ggc      768
Ala Ser Ser Tyr Ser Lys Asn Phe Gly Leu Tyr Asn Glu Arg Val Gly
                245                 250                 255

gct tgt act ctg gtt gct gcc gac agt gaa acc gtt gat cgc gca ttc      816
Ala Cys Thr Leu Val Ala Ala Asp Ser Glu Thr Val Asp Arg Ala Phe
            260                 265                 270

agc caa atg aaa gcg gcg att cgc gct aac tac tct aac cca cca gca      864
Ser Gln Met Lys Ala Ala Ile Arg Ala Asn Tyr Ser Asn Pro Pro Ala
        275                 280                 285

cac ggc gct tct gtt gtt gcc acc atc ctg agc aac gat gcg tta cgt      912
His Gly Ala Ser Val Val Ala Thr Ile Leu Ser Asn Asp Ala Leu Arg
    290                 295                 300

gcg att tgg gaa caa gag ctg act gat atg cgc cag cgt att cag cgt      960
Ala Ile Trp Glu Gln Glu Leu Thr Asp Met Arg Gln Arg Ile Gln Arg
305                 310                 315                 320

atg cgt cag ttg ttc gtc aat acg ctg cag gaa aaa ggc gca aac cgc     1008
Met Arg Gln Leu Phe Val Asn Thr Leu Gln Glu Lys Gly Ala Asn Arg
                325                 330                 335

gac ttc agc ttt atc atc aaa cag aac ggc atg ttc tcc ttc agt ggc     1056
Asp Phe Ser Phe Ile Ile Lys Gln Asn Gly Met Phe Ser Phe Ser Gly
            340                 345                 350

ctg aca aaa gaa caa gtg ctg cgt ctg cgc gaa gag ttt ggc gta tat     1104

Leu Thr Lys Glu Gln Val Leu Arg Leu Arg Glu Glu Phe Gly Val Tyr
        355                 360                 365

gcg gtt gct tct ggt cgc gta aat gtg gcc ggg atg aca cca gat aac 1152
Ala Val Ala Ser Gly Arg Val Asn Val Ala Gly Met Thr Pro Asp Asn
    370                 375                 380

atg gct ccg ctg tgc gaa gcg att gtg gca gtg ctg taa 1191
Met Ala Pro Leu Cys Glu Ala Ile Val Ala Val Leu
385                 390                 395

<210> 20
<211> 396
<212> PRT
<213> Escherichia coli

<400> 20

Met Phe Glu Asn Ile Thr Ala Ala Pro Ala Asp Pro Ile Leu Gly Leu
1               5                   10                  15

Ala Asp Leu Phe Arg Ala Asp Glu Arg Pro Gly Lys Ile Asn Leu Gly
            20                  25                  30

Ile Gly Val Tyr Lys Asp Glu Thr Gly Lys Thr Pro Val Leu Thr Ser
        35                  40                  45

Val Lys Lys Ala Glu Gln Tyr Leu Leu Glu Asn Glu Thr Thr Lys Asn
    50                  55                  60

Tyr Leu Gly Ile Asp Gly Ile Pro Glu Phe Gly Arg Cys Thr Gln Glu
65                  70                  75                  80

Leu Leu Phe Gly Lys Gly Ser Ala Leu Ile Asn Asp Lys Arg Ala Arg
                85                  90                  95

Thr Ala Gln Thr Pro Gly Gly Thr Gly Ala Leu Arg Val Ala Ala Asp
            100                 105                 110

Phe Leu Ala Lys Asn Thr Ser Val Lys Arg Val Trp Val Ser Asn Pro
        115                 120                 125

Ser Trp Pro Asn His Lys Ser Val Phe Asn Ser Ala Gly Leu Glu Val

    130                 135                 140

Arg Glu Tyr Ala Tyr Tyr Asp Ala Glu Asn His Thr Leu Asp Phe Asp
145                 150                 155                 160

Ala Leu Ile Asn Ser Leu Asn Glu Ala Gln Ala Gly Asp Val Val Leu
                165                 170                 175

Phe His Gly Cys Cys His Asn Pro Thr Gly Ile Asp Pro Thr Leu Glu
            180                 185                 190

Gln Trp Gln Thr Leu Ala Gln Leu Ser Val Glu Lys Gly Trp Leu Pro
        195                 200                 205

Leu Phe Asp Phe Ala Tyr Gln Gly Phe Ala Arg Gly Leu Glu Glu Asp
    210                 215                 220

Ala Glu Gly Leu Arg Ala Phe Ala Ala Met His Lys Glu Leu Ile Val
225                 230                 235                 240

Ala Ser Ser Tyr Ser Lys Asn Phe Gly Leu Tyr Asn Glu Arg Val Gly
                245                 250                 255

Ala Cys Thr Leu Val Ala Ala Asp Ser Glu Thr Val Asp Arg Ala Phe
            260                 265                 270

Ser Gln Met Lys Ala Ala Ile Arg Ala Asn Tyr Ser Asn Pro Pro Ala
        275                 280                 285

His Gly Ala Ser Val Val Ala Thr Ile Leu Ser Asn Asp Ala Leu Arg
    290                 295                 300

Ala Ile Trp Glu Gln Glu Leu Thr Asp Met Arg Gln Arg Ile Gln Arg
305                 310                 315                 320

Met Arg Gln Leu Phe Val Asn Thr Leu Gln Glu Lys Gly Ala Asn Arg
                325                 330                 335

Asp Phe Ser Phe Ile Ile Lys Gln Asn Gly Met Phe Ser Phe Ser Gly

            340                 345                 350

Leu Thr Lys Glu Gln Val Leu Arg Leu Arg Glu Glu Phe Gly Val Tyr
        355                 360                 365

Ala Val Ala Ser Gly Arg Val Asn Val Ala Gly Met Thr Pro Asp Asn
    370                 375                 380

Met Ala Pro Leu Cys Glu Ala Ile Val Ala Val Leu
385                 390                 395